(19) 

**Europäisches Patentamt**
**European Patent Office**
**Office européen des brevets**

(11) **EP 3 831 365 A1**

(12) **EUROPEAN PATENT APPLICATION**
published in accordance with Art. 153(4) EPC

(43) Date of publication:
**09.06.2021 Bulletin 2021/23**

(21) Application number: **19845250.0**

(22) Date of filing: **01.08.2019**

(51) Int Cl.:
*A61K 9/14* (2006.01)    *A61K 47/02* (2006.01)
*A61K 31/7088* (2006.01)    *A61P 37/08* (2006.01)
*A61P 11/06* (2006.01)    *C12N 15/113* (2010.01)
*C01B 33/12* (2006.01)

(86) International application number:
**PCT/KR2019/095028**

(87) International publication number:
**WO 2020/027641 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **03.08.2018 US 201862714628 P**

(71) Applicant: **Lemonex Inc.**
**Seoul 08826 (KR)**

(72) Inventor: **WON, Cheol Hee**
**Seoul 08741 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **PHARMACEUTICAL COMPOSITION FOR PREVENTING OR TREATING ATOPIC DISEASES**

(57) The present invention relates to a composition capable of preventing or treating, with excellent efficiency, atopic diseases, such as bronchial asthma, allergic rhinitis, atopic dermatitis, allergic dermatitis or inflammatory skin diseases by effectively inhibiting the expression level of TSLP.

[FIG. 1]

**SLIGRL peptide synthesis**

| Peptide Sequence: | SLIGRL |
|---|---|
| Number of Residues: | 6 |
| N-terminus: | H |
| C-terminus: | NH$_2$ |
| Average Weight: | 656.83 |
| Monoisotopic Weight: | 656.44 |

EP 3 831 365 A1

**Description**

[Technical Field]

The present invention relates to a pharmaceutical composition having good effects of preventing or treating atopic diseases.

[Background Art]

Atopic dermatitis is an intractable disease with chronic itching and skin inflammatory symptoms. The major difference in the clinical manifestations of atopic dermatitis and urticaria is due to a difference in mechanisms causing itching. In general, itching in urticaria is mediated by histamine secreted from mast cells, thus being treated with antihistamine. However, itching of atopic dermatitis is mediated by various immune inflammatory reactions in addition to histamine, such that antihystamine alone cannot effectively control itchy symptoms. Therefore, for effective treatment of atopic diseases, it is very important to heal the itch mediated by the immune response.

Thymic stromal lymphopoietin (TSLP) is greatly secreted by keratinocytes as dermal epithelial cells due to a chronic inflammatory response of atopic diseases, and is an important factor mediating the progression of chronic atopic dermatitis patients to complex asthma disease patients. In addition, TSLP secreted from dermal epithelial cells activates TRPA-1 positive-sensory neurons, causing itching. Although all of the detailed functions of TSLP are not known in the art, recent studies have shown that the expression of TSLP expression is associated with atopic diseases such as atopic dermatitis and/or asthma disease in aspects of pathology, immunology and molecular biology. Therefore, TSLP is considered as a cosmetic, therapeutic, and pharmaceutical important factor.

In order to inhibit TSLP expression, antisense nucleic acids may be used, or a method known as intervention of RNA may be used, such as a chemical treatment method. In addition, double-stranded RNA (dsRNA) oligonucleotides and siRNA oligonucleotides may be used.

Meanwhile, cationic polymers, lipid nanoparticles (LNP), viruses and various nanomaterials have been developed for delivery of siRNAs up to now. The clinical application of cationic polymers and LNPs should be prudent due to toxicity and/or instability of the structure *in vivo,* and viral gene transfer has a problem of mutagenesis in addition to low packaging capacity. Chemical modifications of siRNA backbones may increase stability and cell uptake, but still have disadvantages such as high costs, labor intensive and time consuming processing, and high amounts of siRNA administration for satisfactory efficacy in target cells.

[Summary of Invention]

[Problems to be Solved by Invention]

An object of the present invention is to provide a composition with high efficiency of inhibiting TSLP expression and good effects of preventing or treating atopic diseases.

[Means for Solving Problems]

To achieve the above objects, the following technical solutions are adopted in the present invention.

1. A pharmaceutical composition for preventing or treating atopic diseases, including:

porous silica particles carrying nucleic acid molecules that complementarily bind to at least a portion of TSLP mRNA,
wherein the porous silica particles are characterized in that t, at which an absorbance ratio in the following Equation 1 becomes 1/2, is 24 or more,

[Equation 1]

$$A_t/A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa,

15 ml of the same solvent as the suspension comes into contact with an outside of the permeable membrane, and the inside/outside of the permeable membrane are horizontally stirred at 60 rpm and at 37 °C, pH of the suspension is 7.4, and
$A_t$ indicates absorbance of the porous silica particle measured after lapse of "t" hours since $A_o$ was measured).

2. The pharmaceutical composition according to the above 1, wherein the porous silica particles are prepared by: reacting the silica particles having pores of less than 5 nm in diameter with a swelling agent at 120 to 180 °C for 24 to 96 hours to expand the pores of less than 5 nm in diameter; and calcining the pores of the expanded silica particles at a temperature of 400 °C or higher for 3 hours or more.

3. The pharmaceutical composition according to the above 1, wherein an average diameter of the porous silica particles ranges from 150 to 1000 nm, a BET surface area ranges from 200 to 700 m$^2$/g, and a volume per gram ranges from 0.7 to 2.2 ml.

4. The pharmaceutical composition according to the above 1, wherein the nucleic acid molecule is one of siRNA, dsRNA, PNA or miRNA.

5. The pharmaceutical composition according to the above 4,
wherein the nucleic acid molecules includes at least one siRNA or dsRNA selected from the group consisting of: siRNA composed of a sense RNA having a sequence of SEQ ID NO: 1 and an antisense RNA having a sequence of SEQ ID NO: 47; dsRNA composed of a strand having a sequence of SEQ ID NO: 24 and another strand complementary thereto; siRNA composed of a sense RNA having a sequence of SEQ ID NO: 2 and an antisense RNA having a sequence of SEQ ID NO: 48; dsRNA composed of a strand having a sequence of SEQ ID NO: 25 and another strand complementary thereto; siRNA composed of a sense RNA having a sequence of SEQ ID NO: 3 and an antisense RNA having a sequence of SEQ ID NO: 49; dsRNA composed of a strand having a sequence of SEQ ID NO: 26 and another strand complementary thereto; siRNA composed of a sense RNA having a sequence of SEQ ID NO: 4 and an antisense RNA having a sequence of SEQ ID NO: 50; dsRNA composed of a strand having a sequence of SEQ ID NO: 27 and another strand complementary thereto; siRNA composed of a sense RNA having a sequence of SEQ ID NO: 5 and an antisense RNA having a sequence of SEQ ID NO: 51; dsRNA composed of a strand having a sequence of SEQ ID NO: 28 and another strand complementary thereto; siRNA composed of a sense RNA having a sequence of SEQ ID NO: 6 and an antisense RNA having a sequence of SEQ ID NO: 52; dsRNA composed of a strand having a sequence of SEQ ID NO: 29 and another strand complementary thereto; siRNA composed of a sense RNA having a sequence of SEQ ID NO: 7 and an antisense RNA having a sequence of SEQ ID NO: 53; dsRNA composed of a strand having a sequence of SEQ ID NO: 30 and another strand complementary thereto; siRNA composed of a sense RNA having a sequence of SEQ ID NO: 8 and an antisense RNA having a sequence of SEQ ID NO: 54; dsRNA composed of a strand having a sequence of SEQ ID NO: 31 and another strand complementary thereto; siRNA composed of a sense RNA having a sequence of SEQ ID NO: 9 and an antisense RNA having a sequence of SEQ ID NO: 55; dsRNA composed of a strand having a sequence of SEQ ID NO: 32 and another strand complementary thereto; siRNA composed of a sense RNA having a sequence of SEQ ID NO: 10 and an antisense RNA having a sequence of SEQ ID NO: 56; dsRNA composed of a strand having a sequence of SEQ ID NO: 33 and another strand complementary thereto; siRNA composed of a sense RNA having a sequence of SEQ ID NO: 11 and an antisense RNA having a sequence of SEQ ID NO: 57; dsRNA composed of a strand having a sequence of SEQ ID NO: 34 and another strand complementary thereto; siRNA composed of a sense RNA having a sequence of SEQ ID NO: 12 and an antisense RNA having a sequence of SEQ ID NO: 58; dsRNA composed of a strand having a sequence of SEQ ID NO: 35 and another strand complementary thereto; siRNA composed of a sense RNA having a sequence of SEQ ID NO: 13 and an antisense RNA having a sequence of SEQ ID NO: 59; dsRNA composed of a strand having a sequence of SEQ ID NO: 36 and another strand complementary thereto; siRNA composed of a sense RNA having a sequence of SEQ ID NO: 14 and an antisense RNA having a sequence of SEQ ID NO: 60; dsRNA composed of a strand having a sequence of SEQ ID NO: 37 and another strand complementary thereto; siRNA composed of a sense RNA having a sequence of SEQ ID NO: 15 and an antisense RNA having a sequence of SEQ ID NO: 61; dsRNA composed of a strand having a sequence of SEQ ID NO: 38 and another strand complementary thereto; siRNA composed of a sense RNA having a sequence of SEQ ID NO: 16 and an antisense RNA having a sequence of SEQ ID NO: 62; dsRNA composed of a strand having a sequence of SEQ ID NO: 39 and another strand complementary thereto; siRNA composed of a sense RNA having a sequence of SEQ ID NO: 17 and an antisense RNA having a sequence of SEQ ID NO: 63; dsRNA composed of a strand having a sequence of SEQ ID NO: 40 and another strand complementary thereto; siRNA composed of a sense RNA having a sequence of SEQ ID NO: 18 and an antisense RNA having a sequence of SEQ ID NO: 64; dsRNA composed of a strand having a sequence of SEQ ID NO: 41 and another strand complementary thereto; siRNA composed of a sense RNA having a sequence of SEQ ID NO: 19 and an antisense RNA having a sequence of SEQ ID NO: 65; dsRNA composed of a strand having a sequence of SEQ ID NO: 42 and another strand complementary thereto; siRNA composed of a sense RNA having a sequence of SEQ ID NO: 20 and an antisense RNA having a sequence of SEQ ID NO: 66; dsRNA composed of a strand having a sequence of SEQ ID NO: 43 and another strand complementary thereto; siRNA composed of a sense RNA having a sequence of SEQ ID NO: 21 and an antisense RNA having a sequence of SEQ ID NO: 67; dsRNA composed of a strand having a sequence of SEQ ID NO: 44 and another strand complementary thereto; siRNA composed of a sense RNA having a sequence of SEQ ID NO: 22 and an antisense RNA having a sequence of SEQ ID NO: 68; dsRNA composed of a strand having a sequence of SEQ ID NO: 45 and another strand complementary thereto; siRNA composed of a sense RNA having a sequence of SEQ ID NO: 23 and an antisense RNA having a sequence of SEQ ID NO: 69; and dsRNA composed of a strand having a sequence of SEQ ID NO: 46 and another strand complementary thereto.

6. The pharmaceutical composition according to the above 5, further including a sequence of UU at 3'-terminals of the sense RNA and the antisense RNA sequence.

7. The pharmaceutical composition according to the above 5, further including a sequence of dTdT at 3'-terminals of the sense RNA and the antisense RNA sequence.

8. The pharmaceutical composition according to the above 5, wherein the nucleic acid molecule is at least one siRNA or dsRNA selected from the group consisting of: siRNA composed of a sense RNA having the sequence of SEQ ID NO: 1 and an antisense RNA having the sequence of SEQ ID NO: 47; dsRNA composed of a strand having the sequence of SEQ ID NO: 24 and another strand complementary thereto; siRNA composed of a sense RNA having the sequence of SEQ ID NO: 14 and an antisense RNA having the sequence of SEQ ID NO: 60; dsRNA composed of a strand having the sequence of SEQ ID NO: 37 and another strand complementary thereto; siRNA composed of a sense RNA having the sequence of SEQ ID NO: 21 and an antisense RNA having the sequence of SEQ ID NO: 67; and dsRNA composed of a strand having a sequence of SEQ ID NO: 44 and another strand complementary thereto.

9. The pharmaceutical composition according to the above 8, wherein the nucleic acid molecule include the siRNA composed of a sense RNA having the sequence of SEQ ID NO: 1 and an antisense RNA having the sequence of SEQ ID NO: 47, or the dsRNA composed of a strand having the sequence of SEQ ID NO: 24 and a complementary thereof.

10. The pharmaceutical composition according to the above 1, wherein the porous silica particles are positively charged at neutral pH on an outer surface thereof or an inside of the pores.

11. The pharmaceutical composition according to the above 1, wherein the porous silica particles have hydrophilic or hydrophobic functional groups.

12. The pharmaceutical composition according to the above 1, wherein the atopic disease is at least one selected from the group consisting of bronchial asthma, allergic rhinitis, urticaria, atopic dermatitis, allergic conjunctivitis, allergic dermatitis, allergic contact dermatitis, inflammatory skin disease, pruritus and food allergy.

[Advantageous Effects]

The composition of the present invention may deliver a nucleic acid molecule capable of effectively inhibiting TSLP expression with high efficiency in a sustained manner so as to inhibit TSLP expression with excellent efficiency, thus exhibiting effects of preventing or treating various atopic diseases due to TSLP overexpression.

[Brief Description of Drawings]

FIG. 1 is diagrams illustrating MS analysis values of the synthesized SLIGRL peptide.
FIG. 2 is micrographs of porous silica particles according to one embodiment of the present invention.
FIG. 3 is micrographs of porous silica particles according one embodiment of the present invention.
FIG. 4 is micrographs of small pore particles obtained in a manufacturing process of the porous silica particles according to one embodiment of the present invention.
FIG. 5 is micrographs of the small pore particles according to one embodiment of the present invention.
FIG. 6 is micrographs of the porous silica particles for each pore diameter according to one embodiment of the present invention.

DDV (Degradable Delivery Vehicle) is the particles according to an embodiment, wherein the number in parenthesis means the diameter of the particle and the number of subscripts means the pore diameter. For example, $DDV(200)_{10}$ refers to a particle having a particle diameter (that is, particle size) of 200 nm and a pore diameter of 10 nm according to an embodiment.

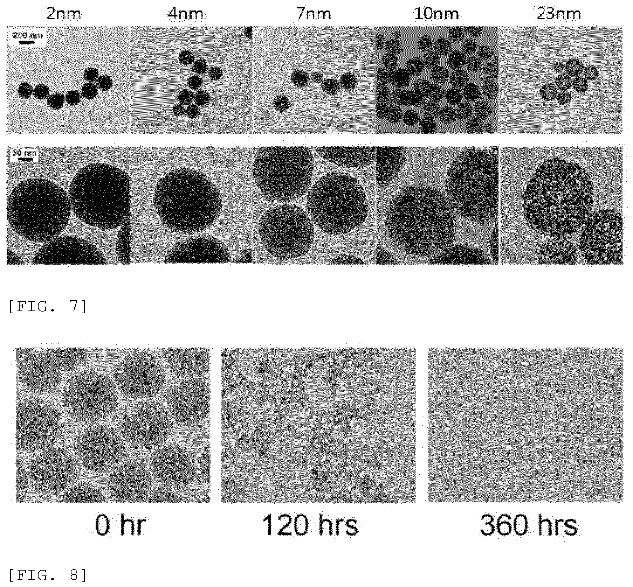

FIG. 7 is micrographs to identify biodegradability of the porous silica particles according to one embodiment of the present invention.

FIG. 8 is a view illustrating a tube having a cylindrical permeable membrane according to one illustrative example.

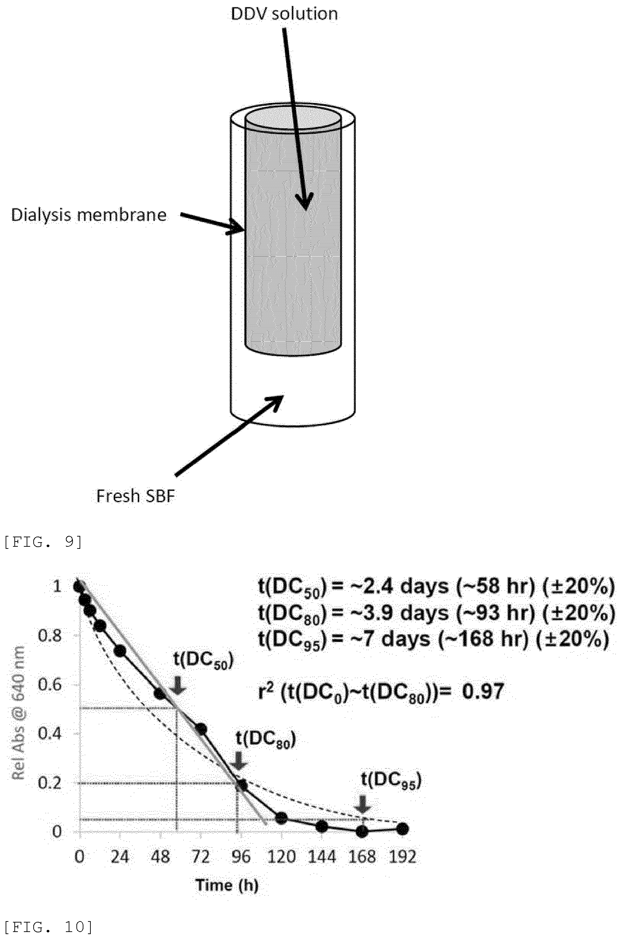

FIG. 9 is a graph illustrating results of decreasing absorbance of the porous silica particles over time according to one embodiment of the present invention.

FIG. 10 is diagrams illustrating results of decreasing absorbance of the porous silica particles for each particle size over time according to one embodiment of the present invention.

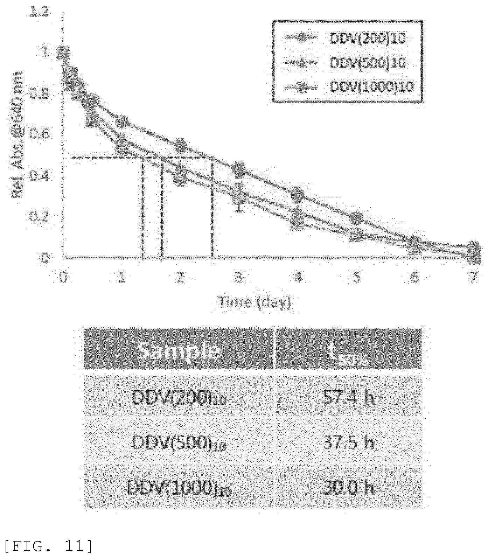

FIG. 11 is diagrams illustrating results of decreasing absorbance of the porous silica particles for each pore diameter over time according to one embodiment of the present invention.

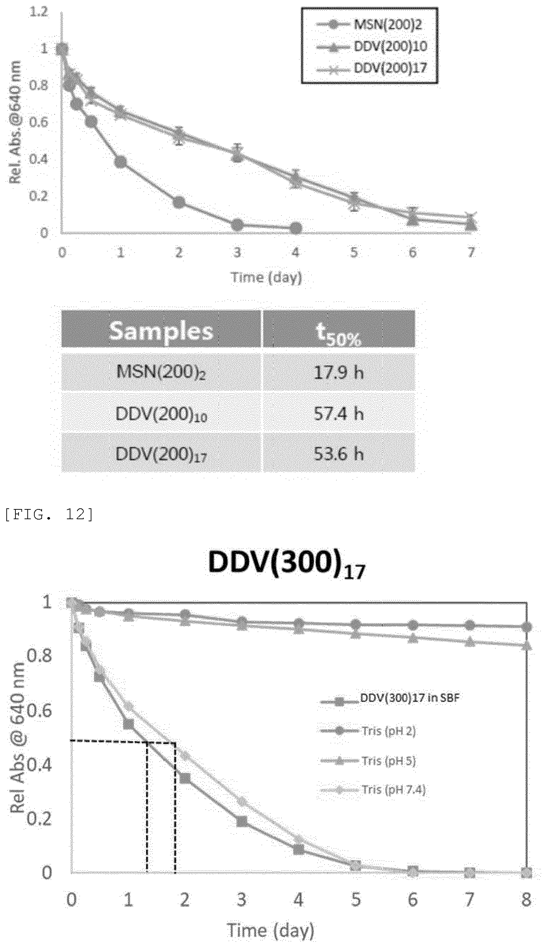

FIG. 12 is a graph illustrating results of decreasing absorbance of the porous silica particles for each pH of the environment over time according to one embodiment of the present invention.

FIG. 13 is a graph illustrating results of decreasing absorbance of the porous silica particles over time according to one embodiment of the present invention.

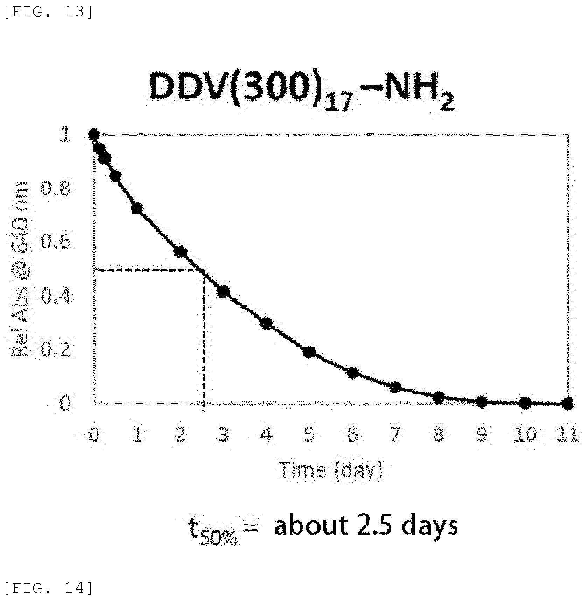

FIG. 14 is a view illustrating a tube to identify siRNA or dsRNA release according to one illustrative example.

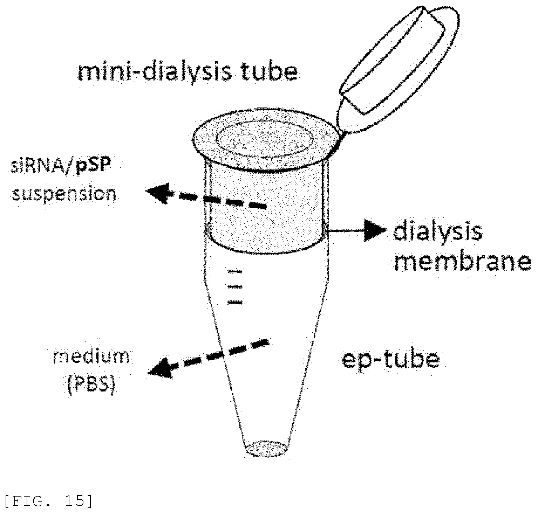

FIG. 15 is a graph illustrating a degree of release of siRNA supported on the porous silica particles over time according to one embodiment of the present invention.

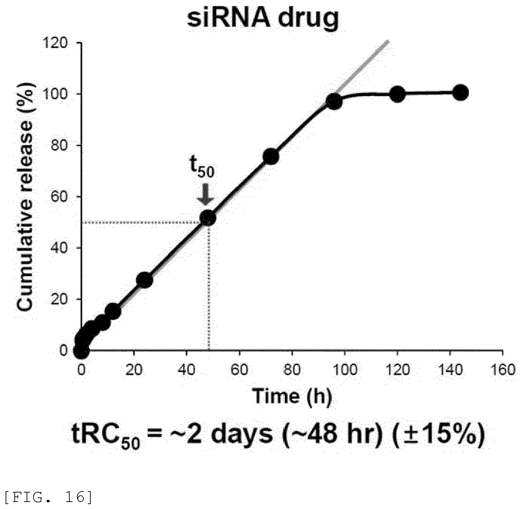

FIG. 16 shows morphological features in HaCaT cells of DDV loaded with siRNA.

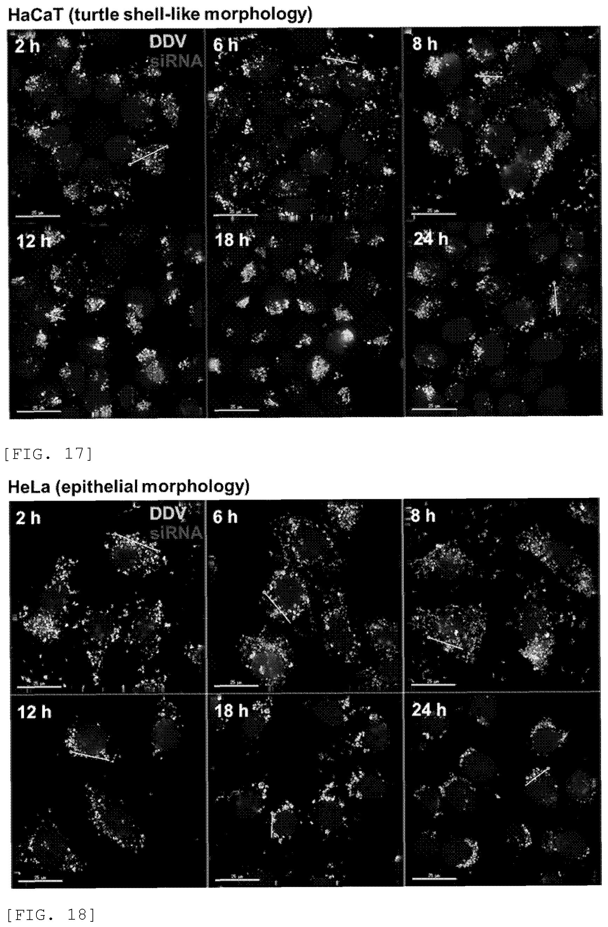

FIG. 17 shows morphological features in HeLa cells of DDV loaded with siRNA.

FIG. 18 illustrates that TSLP expression is induced upon SLIGRL treatment in HaCaT cells.

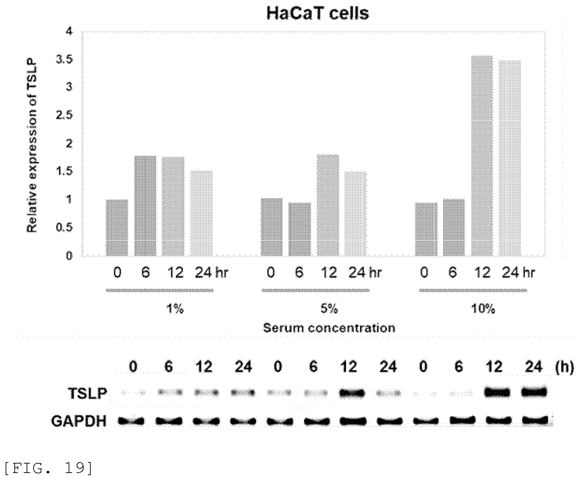

FIG. 19 is a graph illustrating results of identifying TSLP gene expression level through RT-PCR after treatment of HaCaT cells with LEM-siTSLP #1, LEM-siTSLP #14 and LEM-siTSLP #21, followed by SLIGRL treatment to induce TSLP, 12 hours before harvesting.

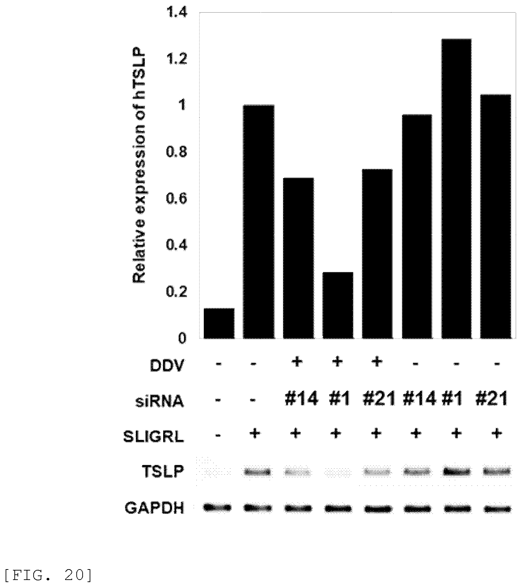

FIG. 20 is a graph illustrating results of comparing TSLP gene expression levels by treating HaCaT cells with LNPs carrying LEM-siTSLP and siTSLP, respectively.

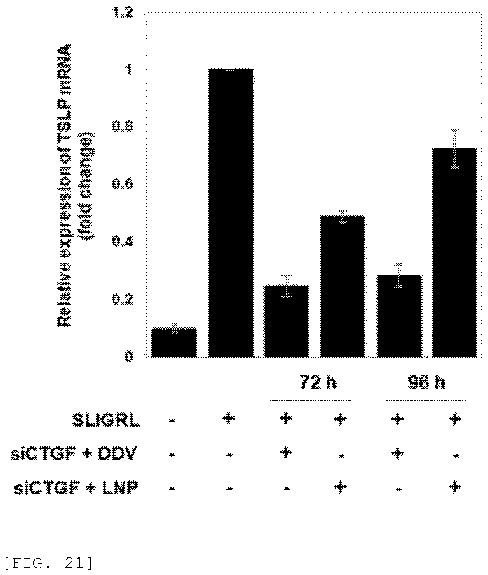

FIG. 21 is a fluorescent image obtained by injecting LEM-siTSLP (mouse) into the skin of a mouse and extracting the skin.

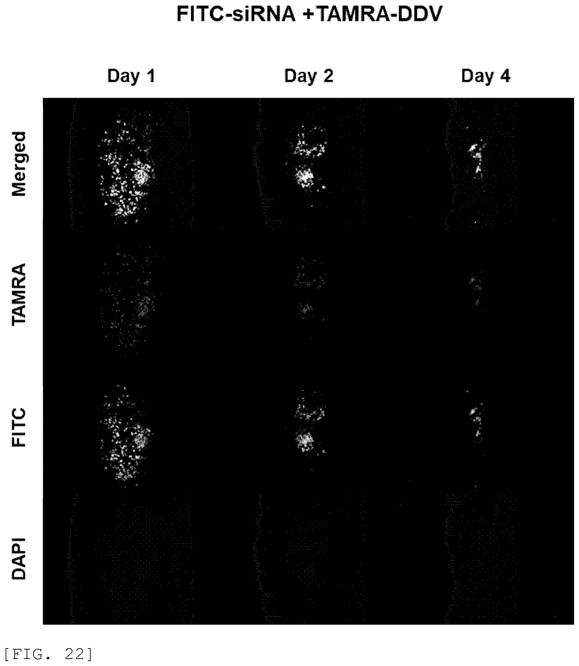

FIG. 22 is a fluorescent image obtained by injecting FITC-conjugated siTSLP (mouse) not loaded in DegradaBALL into the skin of a mouse and extracting the skin.

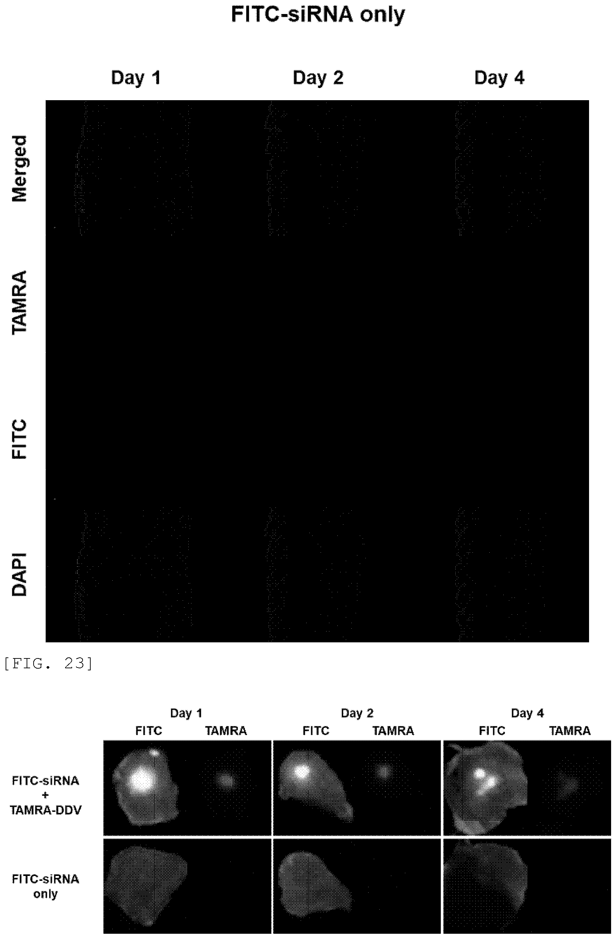

FIG. 23 is a fluorescent image obtained by injecting LEM-siTSLP (mouse) into the skin of a mouse, extracting the skin and determining LEM-siTSLP (mouse) delivery effects as well as a change in distribution.

FIG. 24 is a graph illustrating results of scratching behavior analysis of mice injected with LEM-siTSLP (mouse).

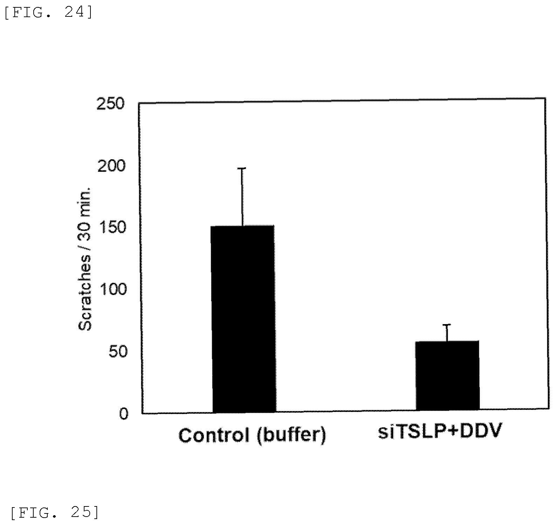

FIG. 25 is a graph illustrating results of scratching behavior analysis of mice injected with LEM-siTSLP (mouse).

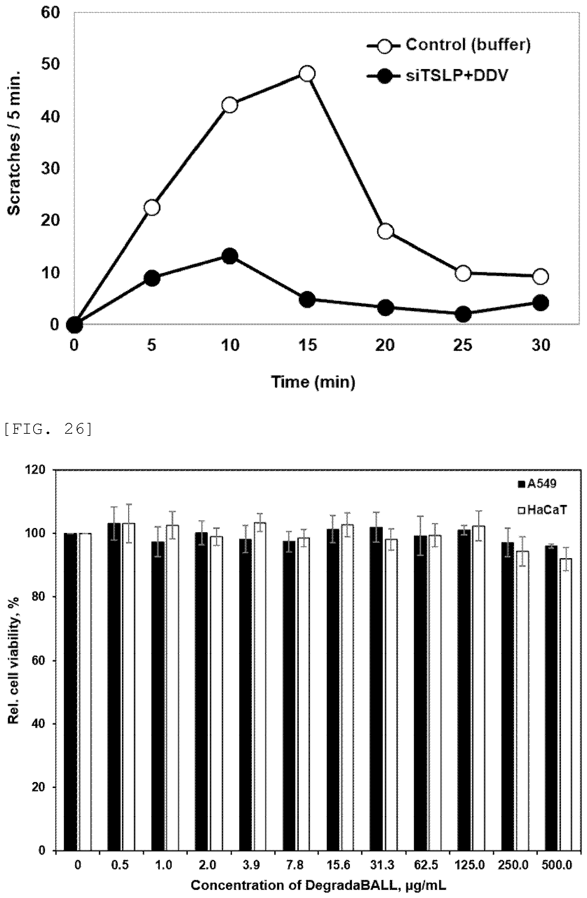

FIG. 26 is a graph illustrating experimental results of observing cell viability after treatment with DegradaBALL.

[Mode for Carrying out Invention]

In the detailed description of the present invention, specific meanings of terms are defined, however, substantially accepted as common meanings understood by those skilled in the art and not intended to be limited to the specific meanings defined below.

"siRNA" refers to a nucleic acid molecule capable of mediating RNA interference or gene silencing. siRNA can suppress expression of a target gene and thus is provided as an efficient gene knockdown method or gene therapy method. The siRNA molecule may have a structure in which a sense strand (a sequence corresponding to mRNA sequence of a target gene) and an antisense strand (a sequence complementary to the mRNA sequence of the target gene) are positioned opposite to each other to form a double-chain. Further, the siRNA molecule may have a single chain structure with self-complementary sense and antisense strands. siRNA is not limited to the complete pairing of double-stranded RNA portions wherein RNAs are paired, but may include impaired portions due to mismatch (the corresponding bases are not complementary), bulge (without bases corresponding to one chain), etc. The siRNA terminal structure may be either blunt or cohesive, as long as the expression of the target gene may be suppressed by RNAi (RNA interference) effects. The cohesive terminal structure may be both a 3'-terminal protrusion structure and a 5'-terminal protrusion structure. Further, the siRNA molecule may include a short nucleotide sequence (e.g., about 5-15 nt) inserted between self-complementary sense and antisense strands. In this case, the siRNA molecule formed by expression of the nucleotide sequence may form a hairpin structure by intramolecular hybridization and form a stem-and-loop structure as a whole. The stem-and-loop structure may be processed *in vitro* or *in vivo* to produce active siRNA molecules capable of mediating RNAi.

"dsRNA" is to a precursor molecule of siRNA, meets RISC complex containing DICER enzyme (Ribonuclease III) of a target cell, and is cleaved into siRNA. In this process, RNAi occurs. dsRNA has a sequence longer by several nucleotides than siRNA, and may have a structure in which a sense strand (a sequence corresponding to mRNA sequence of a target gene) and an antisense strand (a sequence complementary to the mRNA sequence of the target gene) are positioned opposite to each other to form a double-chain.

"PNA" is a synthetic polymer that has a structure similar to DNA or RNA, but is designed to have no charge unlike DNA or RNA thus to have a strong binding force, wherein the DNA and RNA have deoxyribose or ribose backbones, respectively, while a backbone of the PNA has a structure of repeated N-(2-aminoethyl)-glycine ((N-(2-aminoethyl)-glycine) units linked by peptide bonds. The above structure is a structure of purine and pyrimidine bases linked to the backbone by methylene ($-CH_2-$) and carbonyl groups ($-C=O-$), and has N-terminal and C-terminal at both ends thereof similar to peptide.

The term "nucleic acid" means inclusion of any PNA, DNA or RNA, for example, chromosomes, mitochondria, viruses and/or bacterial nucleic acids present in a tissue sample. One or both strands of a double-stranded nucleic acid molecule are included, and any fragment or portion of an intact nucleic acid molecule is also included.

The term "gene" refers to any nucleic acid sequence or a portion thereof that play a functional role in protein coding or transcription or in regulation of other gene expression. The gene may consist of any nucleic acid encoding a functional protein or only a portion of the nucleic acid encoding or expressing protein. A nucleic acid sequence may include gene abnormalities in exons, introns, initial or terminal regions, promoter sequences, other regulatory sequences, or unique sequences adjacent to genes.

The term "gene expression" generally refers to a cellular process in which biologically active polypeptide is produced from a DNA sequence and exhibits biological activity in a cell. In this meaning, the gene expression includes not only transcriptional and translational processes, but also post-transcriptional and post-translational processes that may possibly affect the biological activity of a gene or gene product. The processes include RNA synthesis, processing and transport, as well as polypeptide synthesis, transport and post-translational modifications of polypeptide, but it is not limited thereto. In the case of genes that do not encode protein products, such as siRNA genes, the term "gene expression" refers to a process by which precursor siRNAs are produced from a gene. Typically, this process is referred to as transcription although a transcription product of siRNA gene does not produce a protein by translation, which is different from transcription induced by RNA polymerase II with regard to a protein coding gene. Nevertheless, generation of mature siRNA from siRNA gene is encompassed by the term "gene expression" as that term is used herein.

The term "target gene" refers to a gene that is targeted to be regulated using the methods and compositions as the subject matters disclosed herein. Therefore, the target gene includes a nucleic acid sequence whose expression level is down regulated by siRNA to the mRNA or polypeptide level. Similarly, the term "target RNA" or "target mRNA" refers to the transcript of a target gene to which siRNA is bound to induce regulation of expression of the target gene.

The term "transcription" refers to a cellular process that involves interaction of an expression inducible gene as RNA of structural information present in a coding sequence of the gene with RNA polymerase.

The expression "down-regulation" refers to considerable reduction in expression of specific genes into mRNAs or proteins by intracellular gene transcription or gene translation in activated cells, as compared to normal tissue cells.

The term "treatment" means an approach to obtain beneficial or desirable clinical results. For the purposes of the present invention, beneficial or desirable clinical outcomes include, without limitation thereof, alleviation of symptoms, reduction of disease range, stabilization of disease state (i.e., not worsening), delayed or sustained disease progression, improvement or temporary mitigation and alleviation of disease state (partially or wholly), whether it is detectable or not detected. The term "treatment" may also mean increasing survival compared to expected survival when untreated. The treatment refers to both therapeutic treatment and prophylactic or preventive measures. Such treatment includes not only treatment of disorders to be prevented but also treatment required for already occurring disorders.

The term "prevention" means any action that inhibits or delays development of a relevant disease. It will be apparent to those skilled in the art that the composition of the present invention can prevent initial symptoms, or related diseases if administered before occurrence of the diseases.

Hereinafter, the present invention will be described in detail.

The present invention provides a composition for inhibiting thymic stromal lymphopoietin (TSLP) gene expression; including: porous silica particles carrying nucleic acid molecules that complementarily bind to at least a portion of the transcript of the TSLP gene. The porous silica particles are particles of silica ($SiO_2$) material and have a nano-scaled particle size.

The porous silica nanoparticles of the present invention may include porous particles having nano-sized pores, and may carry the nucleic acid molecules that complementarily bind to at least a portion of TSLP mRNA on a surface of the particles and/or an inside of the pores.

TSLP mRNA of the present invention may be mRNA derived from the same species as the target species, for example, a sequence of SEQ ID NO: 149 for humans, but it is not limited thereto. For example, the TSLP mRNA of the present invention may be human TSLP mRNA, mouse TSLP mRNA, monkey TSLP mRNA, rabbit TSLP mRNA, preferably human TSLP mRNA, but it is not limited thereto.

The nucleic acid molecule of the present invention may be produced differently according to the TSLP mRNA sequences. For example, the nucleic acid molecule of the present invention may be designed to complementarily bind to a human TSLP mRNA sequence, but it is not limited thereto.

The porous silica particles of the present invention are biodegradable particles, which carry a nucleic acid molecule that complementarily binds to at least a portion of TSLP mRNA, and can release the same, that is, the nucleic acid molecule that complementarily binds to at least a portion of TSLP mRNA while being biodegraded in the body when administered to the body. In fact, the porous silica particles of the present invention may be slowly degraded in the body to allow sustained release of the supported nucleic acid molecule that complementarily binds to at least a portion of the TSLP mRNA. For example, "t", at which a ratio of absorbance of the following Equation 1 becomes 1/2, is 24 or more:

[Equation 1]

$$A_t / A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa, 15 ml of the same solvent as the suspension comes into contact with an outside of the permeable membrane, and the inside/outside of the permeable membrane are horizontally stirred at 60 rpm and at 37 °C, pH of the suspension is 7.4, and $A_t$ indicates absorbance of the porous silica particle measured after lapse of "t" hours since $A_0$ was measured).

The above Equation 1 means what a rate the porous silica particles are degraded in an environment similar to the body.

As shown in FIG. 8, for example, absorbances $A_0$ and $A_t$ in the above Equation 1 may be measured after placing porous silica particles and a suspension in a cylindrical permeable membrane and also placing the same suspension outside the permeable membrane.

The porous silica particles of the present invention are biodegradable, and may be slowly degraded in the suspension. The diameter of 50 kDa corresponds to about 5 nm, which allows biodegradable porous silica particles to pass through a permeable membrane having a diameter of 50 kDa, and a cylindrical permeable membrane is under horizontal agitation at 60 rpm to evenly blend the suspension, such that the degraded porous silica particles can come out of the permeable membrane.

The absorbance in the above Equation 1 may be measured, for example, under an environment in which the suspension outside the permeable membrane is replaced with a new suspension. The suspension may be continuously replaced, or replaced every period wherein the period is periodic or irregular. For example, the suspension may be replaced at 1 hour interval, 2 hours interval, 3 hours interval, 6 hours interval, 12 hours interval, 24 hours interval, 2 days interval, 3 days interval, 4 days interval, 7 days interval, etc., within a range of 1 hour to 1 week, but it is not limited thereto.

The absorbance ratio of 1/2 means that the absorbance is half of the initial absorbance after t hours, that is, that approximately half of the porous silica particles are degraded.

The suspension may be a buffer solution, for example, at least one selected from the group consisting of phosphate buffered saline (PBS) and simulated body fluid (SBF), and more specifically, PBS.

"t" in the above Equation 1 of the present invention, at which the absorbance ratio becomes 1/2, may be 24 or more, for example, t may range from 24 to 120. That is, within the above range, t may range from 24 to 96, 24 to 72, 30 to 70, 40 to 70, 50 to 65, etc., but it is not limited thereto.

With regard to the porous silica particles of the present invention, t at which the absorbance ratio in the above Equation 1 becomes 1/5 may range from 70 to 140. For example, t may range from 80 to 140, 80 to 120, 80 to 110, 70 to 140, 70 to 120, 70 to 110, etc. within the above range, but it is not limited thereto.

With regard to the porous silica particles of the present invention, t at which the absorbance ratio in the above Equation 1 becomes 1/20 may range from 130 to 220. For example, t may range from 130 to 200, 140 to 200, 140 to 180, 150 to 180, etc. within the above range, but it is not limited thereto.

With regard to the porous silica particles of the present invention, t at which the absorbance ratio in the above Equation 1 becomes 0.01 or less may be 250 or more. For example, t may be 300 or more, 350 or more, 400 or more, 500 or more, 1000 or more, etc. and the upper limit may be 2000, but it is not limited thereto.

With regard to the porous silica particles of the present invention, the absorbance ratio and t in the above Equation 1 have high positive correlation. For example, Pearson correlation coefficient may be 0.8 or more, and for example, 0.9 or more and 0.95 or more.

"t" in the above Equation 1 means how fast the porous silica particles are degraded under the environment similar to the body. That is, t may be regulated by adjusting, for example, a surface area, a particle size, a pore diameter, substituents on the surface of the porous silica particles and/or the inside of the pores, compactness of the surface and the like.

For example, the surface area of the particles may be increased to reduce t, or the surface area may be decreased to increase t. The surface area may be regulated by adjusting the particle size and the pore diameter of the particles. Further, if direct exposure of the porous silica particles to the environment (such as solvents) is reduced by placing substituents on the surface of the particles and/or the inside of the pores, t may be increased. Further, when the porous silica particles support or carry the nucleic acid molecule that complementarily binds to at least a portion of the TSLP mRNA, and when increasing affinity between the nucleic acid molecule that complementarily binds to at least a portion of the TSLP mRNA and the porous silica particles, direct exposure of the porous silica particles to the environment may be reduced, thereby increasing t. In addition, t may be increased by preparing the particles with more compact surface. As described above, various examples of adjusting t in the above Equation 1 have been described, but it is not limited thereto.

The porous silica particles of the present invention may have a spherical shape, but it is not limited thereto.

The porous silica particles of the present invention may have an average diameter of, for example, 150 to 1000 nm. For example, the average diameter may range from 150 to 800 nm, 150 to 500 nm, 150 to 400 nm, 150 to 300 nm, and 150 to 200 nm, etc. within the above range, but it is not limited thereto.

The porous silica particles of the present invention may have an average pore diameter of, for example, 1 to 100 nm. For example, the pore diameter may range from 5 to 100 nm, 7 to 100 nm, 7 to 50 nm, 10 to 50 nm, 10 to 30 nm, 7 to 30 nm, etc., within the above range, but it is not limited thereto. The porous silica particles having a large diameter as described above may carry a large amount of the nucleic acid molecule that complementarily binds to at least a portion of the TSLP mRNA, and may further carry the nucleic acid molecules that complementarily bind to at least a portion of large-sized TSLP mRNA.

The porous silica particles of the present invention may have a BET surface area of, for example, 200 to 700 $m^2/g$. For example, the BET surface area may range from 200 to 700 $m^2/g$, 200 to 650 $m^2/g$, 250 to 650 $m^2/g$, 300 to 700 $m^2/g$, 300 to 650 $m^2/g$, 300 to 600 $m^2/g$, 300 to 550 $m^2/g$, 300 to 500 $m^2/g$, 300 to 450 $m^2/g$, etc. within the above range, but it is not limited thereto.

Porous silica nanoparticles of the present invention may have a volume per gram, for example, 0.7 to 2.2 ml. For example, the volume may range from 0.7 to 2.0 ml, 0.8 to 2.2 ml, 0.8 to 2.0 ml, 0.9 to 2.0 ml, 1.0 to 2.0 ml, etc. within the above range, but it is not limited thereto. If the volume per gram is too small, a degradation rate may be too high. Further, it is difficult to manufacture excessively large particles or particles having an intact shape.

The porous silica particles of the present invention may have hydrophilic substituents and/or hydrophobic substituents on an outer surface thereof and/or an inside of the pores. For example, only hydrophilic substituents or only hydrophobic substituents may exist on both the surface of the particles and inside of the pores, hydrophilic substituents or hydrophobic substituents may be present on either the surface of the particles or the inside of the pores, or hydrophilic substituents may be present on the surface of the particles while hydrophobic substituents may exist inside of the pores, or vice versa.

Release of the nucleic acid molecule that complementarily binds to at least a portion of the TSLP mRNA supported on the porous silica particles according to the present invention is mainly performed by degradation of nanoparticles. Specifically, interaction of the porous silica particles with the release environment of the nucleic acid molecule that complementarily binds to at least a portion of the TSLP mRNA is adjusted to regulate a degradation rate of the nanoparticles, so that a release rate of the nucleic acid molecule that complementarily binds to at least a portion of the TSLP mRNA may be regulated. Further, the nucleic acid molecule that complementarily binds to at least a portion of the TSLP mRNA may be diffused and released from the nanoparticles, wherein adjusting substituents may regulate a binding force of the nucleic acid molecule that complementarily binds to at least a portion of the TSLP mRNA to the nanoparticles, thereby controlling release of the nucleic acid molecule that complementarily binds to at least a portion of the TSLP mRNA.

Further, in order to increase a binding force of the silica particle to a nucleic acid molecule or material that complementarily binds to at least a portion of poorly soluble (hydrophobic) TSLP mRNA, hydrophobic substituents may be present inside of the pores of the particle. Further, in aspects of easy use and formulation, the surface of the particles may also be treated to have hydrophilic substituents.

The hydrophilic substituents may include, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Further, the hydrophobic substituent may include, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like.

Further, the porous silica particles of the present invention may be positively charged, negatively charged and/or uncharged at an outer surface thereof and/or an inside of the pores. For example, both the surface of the particles and the inside of the pores may be positively charged or negatively charged, only the surface of the particles or the inside of the pores may be positively charged or negatively charged. Alternatively, the surface of the particles may be positively charged while the inside of the pores may be negatively charged or vice versa, which is similar to the case of being uncharged.

The charging may be performed, for example, by the presence of a nonionic substituent, a cationic substituent or an anionic substituent.

The cationic substituent may include, for example, amino group or any other nitrogen-containing group as a basic group, specifically, at least one functional group selected from the group consisting of amino group, aminoalkyl group, alkylamino group, a heterocyclic aromatic compound group containing a nitrogen atom, cyan group and guanidine group, but it is not limited thereto.

The anionic substituent may include, for example, a carboxy group (-COOH), sulfonic acid group (-SO$_3$H), thiol group (-SH), etc. as an acidic group, but it is not limited thereto.

Likewise, when interaction of the porous silica particles with release environment of the nucleic acid molecule that complementarily binds to at least a portion of the TSLP mRNA is regulated by adjusting the substituents through charging, a degradation rate of nanoparticles may be regulated to control a release rate of the nucleic acid molecule that complementarily binds to at least a portion of the TSLP mRNA. Further, the nucleic acid molecule that complementarily binds to at least a portion of the TSLP mRNA may be diffused and released from the nanoparticles. In this regard, adjusting the substituents may regulate a binding force of the nucleic acid molecule that complementarily binds to at least a portion of the TSLP mRNA to the nanoparticles, thereby controlling release of the nucleic acid molecule that complementarily binds to at least a portion of the TSLP mRNA.

Further, the porous silica particles of the present invention may include substituents for the purposes of: supporting the nucleic acid molecule that complementarily binds to at least a portion of the TSLP mRNA on the surface of the particles and/or the inside of the pores; delivery of the nucleic acid molecule that complementarily binds to at least a portion of the TSLP mRNA into a target cell; supporting other substances for other purposes; or binding of additional substituents. Further, the porous silica particles may also include antibodies, ligands, cell permeable peptides, or aptamers bound thereto.

The substituents on the surface of the particles and/or the inside of the pores, charge, binders, etc. described above may be added by, for example, surface modification.

Surface modification may be performed, for example, by reacting a compound having a substituent to be introduced with the particles, wherein the compound may be, for example, alkoxysilane having C1 to C10 alkoxy group, but it is not limited thereto. The alkoxysilane has one or more alkoxy groups, for example, 1 to 3 alkoxy groups. Further, there may be a substituent to be introduced into a site where the alkoxy group is not bound, or a substituent substituted with the same.

The porous silica particles of the present invention may be manufactured, for example, through small pore particle preparation and pore expansion processes and, if necessary, may be manufactured further through calcination, or surface modification process and the like. If both the calcination and the surface modification processes have been implemented, the particles may be surface-modified after calcination.

The small pore particles may be, for example, particles having an average pore diameter of 1 to 5 nm.

The small pore particles may be harvested by adding a surfactant and a silica precursor in a solvent, followed by agitation and homogenization.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and the organic solvent, a relative ratio of water and organic solvent may be, for example, in a volume ratio of 1:0.7 to 1.5, for example, 1:0.8 to 1.3, but it is not limited thereto.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

The surfactant may be added, for example, in an amount of 1 to 10 g, for example, 1 to 8 g, 2 to 8 g or 3 to 8 g per liter of solvent, but it is not limited thereto.

The silica precursor may be added after stirring with addition of a surfactant to the solvent. The silica precursor may be, for example, tetramethyl orthosilicate (TMOS), but it is not limited thereto.

The stirring may be conducted, for example, for 10 to 30 minutes, but it is not limited thereto.

The silica precursor may be added in an amount of 0.5 to 5 ml per liter of solvent, for example, 0.5 ml to 4 ml, 0.5 to 3 ml, 0.5 to 2 ml, 1 to 2 ml, etc. within the above range, but it is not limited thereto.

If necessary, sodium hydroxide may further be used as a catalyst, specifically, and may be added under stirring after addition of the surfactant and before addition of the silica precursor to the solvent.

The sodium hydroxide may be added in an amount of 0.5 to 8 ml per liter of solvent, for example, 0.5 to 5 ml, 0.5 to 4 ml, 1 to 4 ml, 1 to 3 ml, 2 to 3 ml, etc. within the above range with respect to 1 M aqueous sodium hydroxide solution, but it is not thereto.

After addition of the silica precursor, the solution may be reacted with stirring. The stirring may be conducted for 2 to 15 hours, for example, 3 to 15 hours, 4 to 15 hours, 4 to 13 hours, 5 to 12 hours, 6 to 12 hours, 6 to 10 hours, etc. within the above range, but it is not limited thereto. If the stirring time (reaction time) is too short, nucleation may be insufficient.

After agitation, the solution may be aged. Aging may be performed for 8 to 24 hours, for example, for 8 to 20 hours, 8 to 18 hours, 8 to 16 hours, 8 to 14 hours, 10 to 16 hours, 10 to 14 hours, etc. within the above range, but it is not limited thereto.

Thereafter, the reaction product may be washed and dried to harvest porous silica particles and, if necessary, separation of unreacted material may proceed before washing.

Separation of the unreacted material may be implemented by separating the supernatant, for example, through centrifugation. For example, centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the pore of the harvested porous silica particles may be expanded, and such pore expansion may be conducted using a pore swelling agent.

The pore swelling agent may include, for example, trimethylbenzene, triethylbenzene, tripropylbenzene, tributylbenzene, tripentylbenzene, trihexylbenzene, toluene, benzene, etc., and specifically, trimethylbenzene may be used, but it is not limited thereto.

Further, the pore swelling agent used herein may be, for example, N,N-dimethylhexadecylamine (DMHA), but it is not limited thereto.

The pore expansion may be performed, for example, by mixing the porous silica particles in the solvent with a pore swelling agent and heating the mixture to induce reaction.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The porous silica particles may be added in a ratio of 10 to 200 g per liter of solvent, for example, 10 to 150 g, 10 to 100 g, 30 to 100 g, 40 to 100 g, 50 to 100 g, 50 to 80 g, 60 to 80 g, etc., within the above range, but it is not limited thereto.

The porous silica particles may be evenly dispersed in a solvent. For example, the porous silica particles may be added to the solvent and ultrasonically dispersed. In the case of using a mixed solvent, the porous silica particles may be dispersed in a first solvent, followed by adding a second solvent thereto.

The pore swelling agent may be added in a ratio of 10 to 200 parts by volume ("vol. parts") to 100 vol. parts of solvent, for example, 10 to 150 vol. parts, 10 to 100 vol. parts, 10 to 80 vol. parts, 30 to 80 vol. parts, 30 to 70 vol. parts, etc. within the above range, but it is not limited thereto.

The reaction may be carried out at 120 to 190 °C, for example, 120 to 190 °C, 120 to 180 °C, 120 to 170 °C, 130 to 170 °C, 130 to 160 °C, 130 to 150 °C, 130 to 140 °C, etc. within the above range, but it is not limited thereto.

The reaction may be carried out for 6 to 96 hours, for example, 30 to 96 hours, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, 6 to 96 hours, 7 to 96 hours, 8 to 80 hours, 9 to 72 hours, 9 to 80 hours, 6 to 72 hours, 9 to 96 hours, 10 to 80 hours, 10 to 72 hours, 12 to 66 hours, 13 to 60 hours, 14 to 96 hours, 15 to 88 hours, 16 to 80 hours, 17 to 72 hours, etc. within the above range, but it is not limited thereto.

The time and temperature may be desirably adjusted within the above-exemplified range so that the reaction may be carried out sufficiently but not excessively. For example, when the reaction temperature is reduced, the reaction time may be increased, and when the reaction temperature is increased, the reaction time may be shortened. If the reaction is not sufficiently performed, pore expansion may be insufficient. On the other hand, if the reaction proceeds excessively, the particles may collapse due to overexpansion of the pores.

The reaction may be carried out, for example, by gradually raising the temperature. Specifically, the reaction may be carried out by gradually raising the temperature at a rate of 0.5 to 15 °C/min from the room temperature to the above-defined temperature. For example, the temperature may be raised at a rate of 1 to 15 °C/min, 3 to 15 °C/min, 3 to 12 °C/min, 3 to 10 °C/min, etc., but it is not limited thereto.

The reaction may be carried out under stirring. For example, the stirring may be implemented at a speed of 100 rpm or more, and specifically, at a speed of 100 to 1000 rpm, but it is not limited thereto.

After the reaction, the reaction solution may be cooled slowly, for example, by gradually decreasing the temperature. Specifically, the reaction may be carried out by gradually decreasing the temperature at a rate of 0.5 to 20 °C/min from the above-defined temperature to room temperature. For example, the temperature may be decreased at a rate of 1 to 20 °C/min, 3 to 20 °C/min, 3 to 12 °C/min, 3 to 10 °C/min, etc. within the above range, but it is not limited thereto.

After cooling, the reaction product may be washed and dried to harvest porous silica particles having expanded pores. If necessary, unreacted material may be first separated before washing.

Separation of the unreacted material may be implemented by separating the supernatant, for example, through centrifugation. Herein, centrifugation may be conducted, for example, at 6,000 to 10,000 rpm, and the centrifugation time may range from 3 minutes to 60 minutes. For example, the centrifugation may be conducted for 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times, 4 times, 5 times, 6 times, 7 times, 8 times, etc.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the harvested particles may be subjected to calcination, which is a process of heating the particles to remove silanol groups present on the surface of the particles and inside of the pores so as to reduce reactivity of the particles, provide a more compact structure, and remove organic matter filling the pores. For example, the particles may be heated to a temperature of 400 °C or higher. The upper limit of the temperature is not particularly limited but may be 1000 °C, 900 °C, 800 °C, 700 °C, etc. The heating may be conducted, for example, for 3 hours or more, 4 hours or more. The upper limit of the heating time is not particularly limited but may be 24 hours, 12 hours, 10 hours, 8 hours, 6 hours, 5 hours etc. More particularly, the heating may be conducted at 400 to 700 °C for 3 to 8 hours or at 500 to 600 °C for 4 to 5 hours, but it is not limited thereto.

Removing the organic matter filling the pores can prevent some problems of cytotoxicity or foaming caused by the remaining organic matter.

Then, the harvested porous silica particles may be subjected to surface modification, and the surface modification may be performed on the surface of the particles and/or the inside of the pores. Both the particle surface and the inside of the pores may be surface-modified in the same manner, or may be surface-modified differently.

The particles may be charged or have hydrophilic and/or hydrophobic properties through surface modification.

More specifically, in order to effectively support the nucleic acid molecule that complementarily binds to at least a portion of the TSLP mRNA, surface modification of the porous silica particles may be performed by having at least one substituent selected from the group consisting of amino, aminoalkyl, alkylamino, heterocyclic aromatic compound group containing a nitrogen atom, cyan and guanidine groups.

Surface modification may be performed, for example, by reacting a compound having a hydrophilic, hydrophobic, cationic or anionic substituent to be introduced with the particles, wherein the compound may be, for example, alkoxysilane having a C1 to C10 alkoxy group, but it is not limited thereto.

The alkoxysilane has one or more alkoxy groups, for example, 1 to 3 alkoxy groups. Further, there may be a substituent to be introduced into a site where the alkoxy group is not bound, or a substituent substituted with the same.

When alkoxysilane reacts with the porous silica particles, a covalent bond is formed between a silicon atom and an oxygen atom so that the alkoxysilane may be bound to the surface of the porous silica particles and/or the inside of the pores. Since the alkoxysilane has a substituent to be introduced, the corresponding substituent may be introduced into the surface of the porous silica particles and/or the inside of the pores.

The reaction may be carried out by reacting the porous silica particles dispersed in a solvent with alkoxysilane.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The positively charging may be performed by reacting the porous silica particles with alkoxysilane having a basic group such as a nitrogen-containing group, for example, an amino group or an aminoalkyl group. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, 3-(2-aminoethylamino)propyldimethoxymethylsilane, etc. may be used, but it is not limited thereto.

The negatively charging may be performed by reacting the porous silica particles with alkoxysilane having an acidic group such as a carboxyl group, a sulfonic acid group, a thiol group, etc. Specifically, (3-Mercaptopropyl) trimethoxysilane may be used, but it is not limited thereto.

The charging to non-charge (in an uncharged state rather than positive or negative charge) may be performed by reacting the porous silica particles with alkoxysilane having a common functional group having no charge. It is possible to charge with no charge by appropriately combining the positively charging and the negatively charging to offset positive and negative charges, but it is not limited thereto.

The hydrophilic property may be obtained by reacting the porous silica particles with alkoxysilane having a hydrophilic group, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Specifically, N-[3-(trimethoxysilyl)propyl]ethylenediamine, N1-(3-trimethoxysilylpropyl)diethylenetriamine, (3-aminopropyl)trimethoxysilane, (3-mercaptopropyl) trimethoxysilane, trimethoxy[3-(methylamino)propyl]silane, 3-(2-aminoethylamino)propyldimethoxymethylsilane may be used, but it is not limited thereto.

The hydrophobic property may be obtained by reacting the porous silica particles with alkoxysilane having a hydrophobic substituent, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like. Specifically, trimethoxy(octadecyl)silane, trimethoxy-n-octylsilane, trimethoxy(propyl)silane, isobutyl(trimethoxy)silane, trimethoxy(7-octen-1-yl)silane, trimethoxy(3,3,3-trifluoropropyl)silane, trimethoxy(2-phenylethyl)silane, vinyltrimethoxysilane, cyanomethyl, 3-(trimethoxysilyl)propyl]trithiocarbonate, (3-bromopropyl)trimethoxysilane, etc. may be used, but it is not limited thereto.

Further, in order to increase a binding ability of the silica particles to a nucleic acid molecule or material that complementarily binds to at least a portion of poorly soluble (hydrophobic) TSLP mRNA through surface modification, hydrophobic substituents may be present inside of the pores of the particle. Further, in aspects of easy use and formulation, the surface of the particles may also be treated to have hydrophilic substituents. In addition, there may be a substituent on the surface of the particles in order to bind a nucleic acid molecule or material that complementarily binds to at least a portion of another TSLP mRNA.

Further, the surface modification may be performed in combination. For example, surface modification may be performed twice or more on the outer surface of the particles or the inside of the pores. As a specific example, a compound including a carboxyl group may be bound to silica particles having amino groups introduced therein through amide bond in order to change the positively-charged particles to have different surface properties, but it is not limited thereto.

The reaction of the porous silica particles with alkoxysilane may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous silica particles with alkoxysilane may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

The reaction temperature, time and an amount of the compound used for surface modification may be desirably selected according to an extent of surface modification, and reaction conditions will vary depending on hydrophilic property, hydrophobic property and a level of charge with regard to the nucleic acid molecules or materials of the present invention. By controlling the hydrophilic property, hydrophobic property and the level of charge of the porous silica particles, a release rate of the nucleic acid molecules or materials that complementarily bind to at least a portion of the TSLP mRNA may be controlled. For example, if the nucleic acid molecules or materials that complementarily bind to at least a portion of the TSLP mRNA have strong negative charge at neutral pH, the reaction temperature may be raised, the reaction time may be extended or the amount of the treated compound may be increased so as to make the porous silica particles to have strong positive charge, but it is not limited thereto.

Further, the porous silica particles of the present invention may be manufactured through, for example, preparation of small pore particles, pore expansion, surface modification, and internal pore modification.

Preparation of small pore particles and pore expansion may be performed by the above-described processes and, after preparation of the small pore particles and after pore expansion, washing and drying processes may be implemented.

If necessary, unreacted materials may be separated before washing, and separation of the unreacted materials may be conducted by separating the supernatant through centrifugation.

Centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing after preparation of small pore particles may be conducted by a method/condition within the above-described range, but it is not limited thereto.

The washing after pore expansion may be conducted under more moderate conditions than the above embodiments. For example, washing may be conducted three times or less, but it is not limited thereto.

The surface modification and internal pore modification may be performed by the above-described processes, respectively. Herein, surface modification and then internal pore modification may be performed in this order, and a washing process may be further conducted between the above two processes.

When the washing is conducted in more moderated conditions after preparation of small pore particles and pore expansion, a reaction solution such as a surfactant used for particle production and pore expansion is filled in the pores so that the inside of the pores is not modified during surface modification and, instead, only the surface of the particles may be modified. Thereafter, the reaction solution inside of the pores may be washed out and removed.

Particle washing between the surface modification and the internal pore modification processes may be carried out using water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The washing may be carried out under centrifugation, for example at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

The nucleic acid molecule that complementarily binds to at least a portion of the TSLP mRNA may be supported on the surface of the porous silica particles and/or the inside of the pores. Herein, the supporting may be performed, for example, by mixing porous silica particles in a solvent with the nucleic acid molecule that complementarily binds to at least a portion of the TSLP mRNA.

The solvent may be water and/or an organic solvent, and the solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

Further, PBS (phosphate buffered saline solution), SBF (simulated body fluid), borate-buffered saline, tris-buffered saline may be used as the solvent.

A relative ratio of the porous silica particles to the nucleic acid molecules in the present invention is not particularly limited but may be 1:0.05 to 0.8 in weight ratio, for example, 1:0.05 to 0.7, 1:0.05 to 0.6, 1:0.1 to 0.8, 1:0.1 to 0.6, 1:0.2 to 0.8, 1:0.2 to 0.6, etc. within the above range.

The nucleic acid molecule that complementarily binds to at least a portion of the TSLP mRNA supported on the porous silica particles may be gradually released over an extended time. Such sustained release may be continuous or discontinuous, or linear or nonlinear. Further, the release may vary depending upon characteristics of the porous silica particles and/or interaction between the porous silica particles and the nucleic acid molecule that complementarily binds to at least a portion of the TSLP mRNA.

The nucleic acid molecule that complementarily binds to at least a portion of the TSLP mRNA supported on the porous silica particles are released when the porous silica particles are biodegraded. Specifically, the porous silica particles according to the present invention are slowly degraded to allow release of the supported nucleic acid molecule that complementarily binds to at least a portion of the TSLP mRNA in a sustained manner. Such release may be controlled by, for example, adjusting surface area, particle size, pore diameter, substituents on the surface of the particles and/or the inside of the pores, surface compactness, etc. with regard to the porous silica particles, but it is not limited thereto.

The nucleic acid molecule that complementarily binds to at least a portion of the TSLP mRNA supported on the porous silica particles may be released while being separated and diffused from the porous silica particles. Such release is influenced by correlations between the porous silica particles, the nucleic acid molecule that complementarily binds to at least a portion of the TSLP mRNA, and release environment of the same. Therefore, regulating the correlations may control the release of the nucleic acid molecule that complementarily binds to at least a portion of the TSLP mRNA. For example, by enhancing or weakening a binding force of the porous silica particles to the nucleic acid molecule that complementarily binds to at least a portion of the TSLP mRNA through surface modification, the release of the nucleic acid molecule that complementarily binds to at least a portion of the TSLP mRNA may be controlled.

More specifically, in the case where the supported nucleic acid molecule or material that complementarily binds to at least a portion of the TSLP mRNA are poorly soluble (hydrophobic), the surface of the particles and/or the inside of the pores have hydrophobic substituents so as to increase a binding force of the porous silica particles to the nucleic acid molecule or material that complementarily binds to at least a portion of the TSLP mRNA, whereby the nucleic acid molecule or material that complementarily binds to at least a portion of the TSLP mRNA may be released in a sustained manner. For example, the porous silica particles may be surface-modified with alkoxysilane having a hydrophobic substituent.

As used herein, the term "poorly soluble" means to be insoluble, practically insoluble or only slightly soluble (in water), which is a word defined in "Pharmaceutical Science" 18th Edition (issued by U.S.P., Remington, Mack Publishing Company).

The poorly soluble material may have, for example, water solubility of less than 10 g/L, specifically, less than 5 g/L, and more specifically, less than 1 g/L at 1 atmosphere and 25 °C, but it is not limited thereto.

When the supported nucleic acid molecule or material that complementarily binds to at least a portion of the TSLP mRNA is water-soluble (hydrophilic), the surface of the particles and/or the inside of the pores have hydrophilic substituents so as to increase a binding force of the porous silica particles to the nucleic acid molecule or material that complementarily binds to at least a portion of the TSLP mRNA, whereby the nucleic acid molecule or material that complementarily binds to at least a portion of the TSLP mRNA may be released in a sustained manner. For example, the porous silica particles may be surface-modified with alkoxysilane having a hydrophilic substituent.

For example, the water-soluble material may have a water solubility of 10 g/L or more at 1 atmosphere and 25 °C, but it is not limited thereto.

In the case where the supported nucleic acid molecule or material that complementarily binds to at least a portion of the TSLP mRNA is charged, the surface of the particles and/or the inside of the pores are charged with opposite charges, so as to increase the binding force of the porous silica particles to the nucleic acid molecule or material that complementarily binds to at least a portion of the TSLP mRNA, whereby the nucleic acid molecule or material that complementarily binds to at least a portion of the TSLP mRNA may be released in a sustained manner. For example, the porous silica particles may be surface-modified with alkoxysilane having an acidic group or a basic group.

Specifically, if the nucleic acid molecule or material that complementarily binds to at least a portion of the TSLP mRNA is positively charged at neutral pH, the surface of the particles and/or the inside of the pores may be negatively charged at neutral pH so as to increase a binding force of the porous silica particles to the nucleic acid molecule or material that complementarily binds to at least a portion of the TSLP mRNA, whereby the nucleic acid molecule or material that complementarily binds to at least a portion of the TSLP mRNA may be released in a sustained manner. For example, the porous silica particles may be surface-modified with alkoxysilane having an acidic group such as a carboxyl group (-COOH), a sulfonic acid group (-SO$_3$H), etc.

Further, if the nucleic acid molecule or material that complementarily binds to at least a portion of the TSLP mRNA is negatively charged at neutral pH, the surface of the particles and/or the inside of the pores may be positively charged at neutral pH, so as to increase a binding force of the porous silica particles to the nucleic acid molecule or material that complementarily binds to at least a portion of the TSLP mRNA, whereby the nucleic acid molecule or material that complementarily binds to at least a portion of the TSLP mRNA may be released in a sustained manner. For example, the porous silica particles may be surface-modified with alkoxysilane having a basic group such as an amino group or any other nitrogen-containing group.

The nucleic acid molecule or material that complementarily binds to at least a portion of the TSLP mRNA may be released for a period of, for example, 7 days to 1 year or more, depending upon types of treatment to be required, release environments and types of porous silica particles to be used.

Further, since the porous silica particles of the present invention are 100% biodegradable, the nucleic acid molecule or material that complementarily binds to at least a portion of the TSLP mRNA may be 100% released.

The nucleic acid molecule may be a single strand of siRNA, dsRNA, PNA or miRNA and, in this case, the siRNA, dsRNA, PNA or miRNA may inhibit expression of TSLP gene by RNAi (RNA interference). More specifically, the nucleic acid molecule may be complementarily bind to TSLP mRNA at least a portion of the region, thereby inhibiting TSLP gene expression.

Specifically, the nucleic acid molecules may include at least one siRNA or dsRNA selected from the group consisting of: siRNA composed of a sense RNA having a sequence of SEQ ID NO: 1 and an antisense RNA having a sequence of SEQ ID NO: 47; dsRNA composed of a strand having a sequence of SEQ ID NO: 24 and another strand complementary thereto; siRNA composed of a sense RNA having a sequence of SEQ ID NO: 2 and an antisense RNA having a sequence of SEQ ID NO: 48; dsRNA composed of a strand having a sequence of SEQ ID NO: 25 and another strand complementary thereto; siRNA composed of a sense RNA having a sequence of SEQ ID NO: 3 and an antisense RNA having a sequence of SEQ ID NO: 49; dsRNA composed of a strand having a sequence of SEQ ID NO: 26 and another strand complementary thereto; siRNA composed of a sense RNA having a sequence of SEQ ID NO: 4 and an antisense RNA having a sequence of SEQ ID NO: 50; dsRNA composed of a strand having a sequence of SEQ ID NO: 27 and another strand complementary thereto; siRNA composed of a sense RNA having a sequence of SEQ ID NO: 5 and an antisense RNA having a sequence of SEQ ID NO: 51; dsRNA composed of a strand having a sequence of SEQ ID NO: 28 and another strand complementary thereto; siRNA composed of a sense RNA having a sequence of SEQ ID NO: 6 and an antisense RNA having a sequence of SEQ ID NO: 52; dsRNA composed of a strand having a sequence of SEQ ID NO: 29 and another strand complementary thereto; siRNA composed of a sense RNA having a sequence of SEQ ID NO: 7 and an antisense RNA having a sequence of SEQ ID NO: 53; dsRNA composed of a strand having a sequence of SEQ ID NO: 30 and another strand complementary thereto; siRNA composed of a sense RNA having a sequence of SEQ ID NO: 8 and an antisense RNA having a sequence of SEQ ID NO: 54; dsRNA composed of a strand having a sequence of SEQ ID NO: 31 and another strand complementary thereto; siRNA composed of a sense RNA having a sequence of SEQ ID NO: 9 and an antisense RNA having a sequence of SEQ ID NO: 55; dsRNA composed of a strand having a sequence of SEQ ID NO: 32 and another strand complementary thereto; siRNA composed of a sense RNA having a sequence of SEQ ID NO: 10 and an antisense RNA having a sequence of SEQ ID NO: 56; dsRNA composed of a strand having a sequence of SEQ ID NO: 33 and another strand complementary thereto; siRNA composed of a sense RNA having a sequence of SEQ ID NO: 11 and an antisense RNA having a sequence of SEQ ID NO: 57; dsRNA composed of a strand having a sequence of SEQ ID NO: 34 and another strand complementary thereto; siRNA composed of a sense RNA having a sequence of SEQ ID NO: 12 and an antisense RNA having a sequence of SEQ ID NO: 58; dsRNA composed of a strand having a sequence of SEQ ID NO: 35 and another strand complementary thereto; siRNA composed of a sense RNA having a sequence of SEQ ID NO: 13 and an antisense RNA having a sequence of SEQ ID NO: 59; dsRNA composed of a strand having a sequence of SEQ ID NO: 36 and another strand complementary thereto; siRNA composed of a sense RNA having a sequence of SEQ ID NO: 14 and an antisense RNA having a sequence of SEQ ID NO: 60; dsRNA composed of a strand having a sequence of SEQ ID NO: 37 and another strand complementary thereto; siRNA composed of a sense RNA having a sequence of SEQ ID NO: 15 and an antisense RNA having a sequence of SEQ ID NO: 61; dsRNA composed of a strand having a sequence of SEQ ID NO: 38 and another strand complementary thereto; siRNA composed of a sense RNA having a sequence of SEQ ID NO: 16 and an antisense RNA having a sequence of SEQ ID NO: 62; dsRNA composed of a strand having a sequence of SEQ ID NO: 39 and another strand complementary thereto; siRNA composed of a sense RNA having a sequence of SEQ ID NO: 17 and an antisense RNA having a sequence of SEQ ID NO: 63; dsRNA composed of a strand having a sequence of SEQ ID NO: 40 and another strand complementary thereto; siRNA composed of a sense RNA having a sequence of SEQ ID NO: 18 and an antisense RNA having a sequence of SEQ ID NO: 64; dsRNA composed of a strand having a sequence of SEQ ID NO: 41 and another strand complementary thereto; siRNA composed of a sense RNA having a sequence of SEQ ID NO: 19 and an antisense RNA having a sequence of SEQ ID NO: 65; dsRNA composed of a strand having a sequence of SEQ ID NO: 42 and another strand complementary thereto; siRNA composed of a sense RNA having a sequence of SEQ ID NO: 20 and an antisense RNA having a sequence of SEQ ID NO: 66; dsRNA composed of a strand having a sequence of SEQ ID NO: 43 and another strand complementary thereto; siRNA composed of a sense RNA having a sequence of SEQ ID NO: 21 and an antisense RNA having a sequence of SEQ ID NO: 67; dsRNA composed of a strand having a sequence of SEQ ID NO: 44 and another strand complementary thereto; siRNA composed of a sense RNA having a sequence of SEQ ID NO: 22 and an antisense RNA having a sequence of SEQ ID NO: 68; dsRNA composed of a strand having a sequence of SEQ ID NO: 45 and another strand complementary thereto; siRNA composed of a sense RNA having a sequence of SEQ ID NO: 23 and an antisense RNA having a sequence of SEQ ID NO: 69; and dsRNA composed of a strand having a sequence of SEQ ID NO: 46 and another strand complementary thereto, which are listed in Table 1 below, but are not limited thereto.

[TABLE 1]

| Target sequence 1: SEQ ID NO: 126<br>5'-GCA GCC UAU CUC AGU ACU A-3'<br>(Position in gene sequence: 140) | siRNA GC content: 47.37% |
| | Sense strand: SEQ ID NO: 1 5'-GCA GCC UAU CUC AGU ACU A-3' |
| | Antisense strand: SEQ ID NO: 47 5'-UAGUACUGAGAUAGGCUGC-3' |
| | dsRNA: SEQ ID NO: 24<br>5'-GCA GCC UAU CUC AGU ACU AUU UCU A-3' |
| Target sequence 2: SEQ ID NO: 127<br>5'-GCC UAU CUC AGU ACU AUU U-3'<br>(Position in gene sequence: 143) | siRNA GC content: 36.85% |
| | Sense strand: SEQ ID NO: 2 5'-GCC UAU CUC AGU ACU AUU U-3' |
| | Antisense strand: SEQ ID NO: 48 5'-AAA UAG UAC UGA GAU AGG C-3' |
| | dsRNA: SEQ ID NO: 25<br>5'-GCC UAU CUC AGU ACU AUU UCU AAA G-3' |
| Target sequence 3: SEQ ID NO:128<br>5'-GCC ACA UUG CCU UAC UGA A-3'<br>(Position in gene sequence: 235) | siRNA GC content: 47.37% |
| | Sense strand: SEQ ID NO: 3 5'-GCC ACA UUG CCU UAC UGA A-3' |
| | Antisense strand: SEQ ID NO: 49 5'-UUC AGU AAG GCA AUG UGG C-3' |
| | dsRNA: SEQ ID NO: 26<br>5'-GCC ACA UUG CCU UAC UGA AAU CCA G-3' |
| Target sequence 4: SEQ ID NO: 129<br>5'-CCA CAU UGC CUU ACU GAA A-3'<br>(Position in gene sequence: 236) | siRNA GC content: 42.11% |
| | Sense strand: SEQ ID NO: 4 5'-CCA CAU UGC CUU ACU GAA A-3' |
| | Antisense strand: SEQ ID NO: 50 5'-UUU CAG UAA GGC AAU GUG G-3' |
| | dsRNA: SEQ ID NO: 27<br>5'-CCA CAU UGC CUU ACU GAA AUC CAG A-3' |
| Target sequence 5: SEQ ID NO: 130<br>5'-UCC AGA GCC UAA CCU UCA A-3'<br>(Position in gene sequence: 255) | siRNA GC content: 47.37% |
| | Sense strand: SEQ ID NO: 5 5'-UCC AGA GCC UAA CCU UCA A-3' |
| | Antisense strand: SEQ ID NO: 51 5'-UUG AAG GUU AGG CUC UGG A-3' |
| | dsRNA: SEQ ID NO: 28<br>5'-UCC AGA GCC UAA CCU UCA AUC CCC A-3' |
| Target sequence 6: SEQ ID NO: 131<br>5'-CCA GAG CCU AAC CUU CAA U-3'<br>(Position in gene sequence: 256) | siRNA GC content: 47.37% |
| | Sense strand: SEQ ID NO: 6 5'-CCA GAG CCU AAC CUU CAA U-3' |

(continued)

| | |
|---|---|
| | Antisense strand: SEQ ID NO: 52 5'-AUU GAA GGU UAG GCU CUG G-3' |
| | dsRNA: SEQ ID NO: 29<br><br>5'-CCA GAG CCU AAC CUU CAA UCC CAC C-3' |
| Target sequence 7: SEQ ID NO: 132<br><br>5'-GCG UCG CUC GCC AAA GAA A-3'<br>(Position in gene sequence: 290) | siRNA GC content: 52.9% |
| | Sense strand: SEQ ID NO: 7 5'-GCG UCG CUC GCC AAA GAA A-3' |
| | Antisense strand: SEQ ID NO: 53 5'-UUU CUU UGG CGA GCG ACG C-3' |
| | dsRNA: SEQ ID NO: 30<br><br>5'-GCG UCG CUC GCC AAA GAA AUG UUC G-3' |
| Target sequence 8: SEQ ID NO: 133<br><br>5'-CCA AAG AAA UGU UCG CCA U-3'<br>(Position in gene sequence: 300) | siRNA GC content: 42.11% |
| | Sense strand: SEQ ID NO: 8 5'-CCA AAG AAA UGU UCG CCA U-3' |
| | Antisense strand: SEQ ID NO: 54 5'-AUG GCG AAC AUU UCU UUG G-3' |
| | dsRNA: SEQ ID NO: 31<br><br>5'-CCA AAG AAA UGU UCG CCA UGA AAA C-3' |
| Target sequence 9: SEQ ID NO: 134<br><br>5'-GCU UCA AUC GAC CUU UAC U-3'<br>(Position in gene sequence: 468) | siRNA GC content: 42.11% |
| | Sense strand: SEQ ID NO: 9 5'-GCU UCA AUC GAC CUU UAC U-3' |
| | Antisense strand: SEQ ID NO: 55 5'-AGU AAA GGU CGA UUG AAG C-3' |
| | dsRNA: SEQ ID NO: 32<br><br>5'-GCU UCA AUC GAC CUU UAC UGA AAC A-3' |
| Target sequence 10: SEQ ID NO: 135<br><br>5'-UCA AUC GAC CUU UAC UGA A-3'<br>(Position in gene sequence: 471) | siRNA GC content: 36.85% |
| | Sense strand: SEQ ID NO: 10 5'-UCA AUC GAC CUU UAC UGA A-3' |
| | Antisense strand: SEQ ID NO: 56 5'-UUC AGU AAA GGU CGA UUG A-3' |
| | dsRNA: SEQ ID NO: 33<br><br>5'-UCA AUC GAC CUU UAC UGA AAC AAC A-3' |
| Target sequence 11: SEQ ID NO: 136<br><br>5'-GCC UUA CUA UAU GUU CUG UC-3'<br>(Position in gene sequence: 32) | siRNA GC content: 40.00% |
| | Sense strand: SEQ ID NO: 11 5'-GCC UUA CUA UAU GUU CUG UC-3' |
| | Antisense strand: SEQ ID NO: 57 5'-GAC AGA ACA UAU AGU AAG GC-3' |
| | dsRNA: SEQ ID NO: 34<br><br>5'-GCC UUA CUA UAU GUU CUG UCA GUU U-3' |
| | siRNA GC content: 38.10% |

(continued)

| Target sequence 12: SEQ ID NO: 137 5'-CCU UAC UAU AUG UUC UGU CAG-3' (Position in gene sequence: 33) | Sense strand: SEQ ID NO: 12 5'-CCU UAC UAU AUG UUC UGU CAG-3' |
| --- | --- |
| | Antisense strand: SEQ ID NO: 58 5'-CUG ACA GAA CAU AUA GUA AGG-3' |
| | dsRNA: SEQ ID NO: 35 5'-CCU UAC UAU AUG UUC UGU CAG UUU C-3' |
| Target sequence 13: SEQ ID NO: 138 5'-CAG GAA AAU CUU CAU CUU AC-3' (Position in gene sequence: 61) | siRNA GC content: 35.00% |
| | Sense strand: SEQ ID NO: 13 5'-CAG GAA AAU CUU CAU CUU AC-3' |
| | Antisense strand: SEQ ID NO: 59 5'-GUA AGA UGA AGA UUU UCC UG-3' |
| | dsRNA: SEQ ID NO: 36 5'-CAG GAA AAU CUU CAU CUU ACA ACU U-3' |
| Target sequence 14: SEQ ID NO: 139 5'-GCU GGU GUU AAC UUA CGA CU-3' (Position in gene sequence: 91) | siRNA GC content: 45.00% |
| | Sense strand: SEQ ID NO: 14 5'-GCU GGU GUU AAC UUA CGA CU-3' |
| | Antisense strand: SEQ ID NO: 60 5'-AGU CGU AAG UUA ACA CCA GC-3' |
| | dsRNA: SEQ ID NO: 37 5'-GCU GGU GUU AAC UUA CGA CUC UUC A-3' |
| Target sequence 15: SEQ ID NO: 140 5'-GGU GUU AAC UUA CGA CUU CA-3' (Position in gene sequence: 94) | siRNA GC content: 40.00% |
| | Sense strand: SEQ ID NO: 15 5'-GGU GUU AAC UUA CGA CUU CA-3' |
| | Antisense strand: SEQ ID NO: 61 5'-UGA AGU CGU AAG UUA ACA CC-3' |
| | dsRNA: SEQ ID NO: 38 5'-GGU GUU AAC UUA CGA CUU CAC UAA C-3' |
| Target sequence 16: SEQ ID NO: 141 5'-CAC UAA CUG UGA CUU UGA G-3' (Position in gene sequence: 112) | siRNA GC content: 42.11% |
| | Sense strand: SEQ ID NO: 16 5'-CAC UAA CUG UGA CUU UGA G-3' |
| | Antisense strand: SEQ ID NO: 62 5'-CUC AAA GUC ACA GUU AGU G-3' |
| | dsRNA: SEQ ID NO: 39 5'-CAC UAA CUG UGA CUU UGA GAA GAU U-3' |
| Target sequence 17: SEQ ID NO: 142 5'-GAC CUG AUU ACA UAU AUG AG-3' (Position in gene sequence: 167) | siRNA GC content: 35.00% |
| | Sense strand: SEQ ID NO: 17 5'-GAC CUG AUU ACA UAU AUG AG-3' |
| | Antisense strand: SEQ ID NO: 63 5'-CUC AUA UAU GUA AUC AGG UC-3' |
| | 5'-GAC CUG AUU ACA UAU AUG AGU GGG A-3' |

(continued)

| | |
|---|---|
| Target sequence 18: SEQ ID NO: 143<br><br>5'-CCG AGU UCA ACA ACA CCG U-3'<br><br>(Position in gene sequence: 201) | siRNA GC content: 52.63% |
| | Sense strand: SEQ ID NO: 18 5'-CCG AGU UCA ACA ACA CCG U-3' |
| | Antisense strand: SEQ ID NO: 64 5'-ACG GUG UUG UUG AAC UCG G-3' |
| | dsRNA: SEQ ID NO: 41<br><br>5'-CCG AGU UCA ACA ACA CCG UCU CUU G-3' |
| Target sequence 19: SEQ ID NO: 144<br><br>5'-ACC GUC UCU UGU AGC AAU CG-3'<br><br>(Position in gene sequence: 215) | siRNA GC content: 50.00% |
| | Sense strand: SEQ ID NO: 19 5'-ACC GUC UCU UGU AGC AAU CG-3' |
| | Antisense strand: SEQ ID NO: 65 5'-CGA UUG CUA CAA GAG ACG GU-3' |
| | dsRNA: SEQ ID NO: 42<br><br>5'-ACC GUC UCU UGU AGC AAU CGG CCA C-3' |
| Target sequence 20: SEQ ID NO: 145<br><br>5'-AAG GCU GCC UUA GCU AUC UG-3'<br><br>(Position in gene sequence: 326) | siRNA GC content: 50.00% |
| | Sense strand: SEQ ID NO: 20 5'-AAG GCU GCC UUA GCU AUC UG-3' |
| | Antisense strand: SEQ ID NO: 66 5'-CAG AUA GCU AAG GCA GCC UU-3' |
| | dsRNA: SEQ ID NO: 43<br><br>5'-AAG GCU GCC UUA GCU AUC UGG UGC C-3' |
| Target sequence 21: SEQ ID NO: 146<br><br>5'-CGG AAA CUC AGA UAA AUG C-3'<br><br>(Position in gene sequence: 360) | siRNA GC content: 42.11%<br><br>Sense strand: SEQ ID NO: 21 5'-CGG AAA CUC AGA UAA AUG C-3' |
| | Antisense strand: SEQ ID NO: 67 5'-GCA UUU AUC UGA GUU UCC G-3' |
| | dsRNA: SEQ ID NO: 44<br><br>5'-CGG AAA CUC AGA UAA AUG CUA CUC A-3' |
| Target sequence 22: SEQ ID NO: 147<br><br>5'-CCA ATA AAT GTC TGG AAC AA-3'<br><br>(Position in gene sequence: 420) | siRNA GC content: 35.00% |
| | Sense strand: SEQ ID NO: 22 5'-CCA AUA AAU GUC UGG AAC AA-3' |
| | Antisense strand: SEQ ID NO: 68 5'-UUG UUC CAG ACA UUU AUU GG-3' |
| | dsRNA: SEQ ID NO: 45<br><br>5'-CCA ATA AAT GTC TGG AAC AAG UGU C-3' |
| | siRNA GC content: 57.89% |

(continued)

| Target sequence 23: SEQ ID NO: 148 5'-CAA GGA UUG UGG CGU CGC U-3' (Position in gene sequence: 442) | Sense strand: SEQ ID NO: 23 5'-CAA GGA UUG UGG CGU CGC U-3' |
|---|---|
| | Antisense strand: SEQ ID NO: 69 5'-AGC GAC GCC ACA AUC CUU G-3' |
| | dsRNA: SEQ ID NO: 46 5'-CAA GGA UUG UGG CGU CGC UGC UUC A-3' |

Nucleic acid molecules of the present invention may be derived from animals including human, for example, monkeys, pigs, horses, cows, sheep, dogs, cats, mice, rabbits, and the like, and preferably derived from human.

The nucleic acid molecule of the present invention has been modified by deletion, substitution or insertion of functional equivalents of the nucleic acid molecule to constitute the same, for example, some nucleotide sequences of the nucleic acid molecule according to the present invention. However, variants with the same functions as the nucleic acid molecule of the present invention may substantially belong to the same concept.

More specifically, when the nucleic acid molecule of the present invention forms a sense RNA or antisense RNA of siRNA, the sense RNA and antisense RNA sequence may further include a sequence of UU or dTdT at 3'-terminal thereof. In this case, the siRNA or dsRNA may have advantages such as improvement of structural stability of siRNA or dsRNA through an increase in resistance to nucleic acid hydrolase, improvement of RNAi efficiency of siRNA or dsRNA through induction of stable RISC and the like.

Nucleic acid molecules of the present invention may be isolated or prepared using standard molecular biology techniques such as chemical synthesis or recombinant methods, or may be commercially available. Further, the composition of the present invention may include not only the nucleic acid molecule itself of the present invention but also other substances capable of increasing an expression rate of the nucleic acid molecule of the present invention in cells, for example, compounds, natural products, novel proteins and the like..

Meanwhile, the nucleic acid molecule of the present invention may be provided with being included in a vector for intracellular expression.

The nucleic acid molecules of the present invention may be introduced into cells using diverse transformation techniques, such as complexes of DNA and DEAE-dextran, complexes of DNA and nuclear proteins, complexes of DNA and lipids. For this purpose, the nucleic acid molecules of the present invention may be included within a carrier that allows for efficient introduction into a cell. The carrier is preferably a vector, and both viral and non-viral vectors may be used. Viral vectors may include, for example, lentivirus, retrovirus, adenovirus, herpesvirus, abipoxvirus vectors, and the like, and preferably a lentiviral vector, but it is not limited thereto. Lentiviruses are a type of retrovirus with features that can infect undivided cells as well as divided cells due to nucloephilicity of a pre-integrated complex (virus "shell") enabling active introduction into nucleopore or a complete nuclear membrane.

The vector containing the nucleic acid molecule of the present invention preferably further includes a selectable marker. The "selectable marker" is intended to facilitate selection of cells into which the nucleic acid molecule of the present invention is introduced. The selectable markers possibly used in the vector are not particularly limited as long as they are genes capable of easily detecting or determining whether to introduce the vector or not. However, representative selectable markers may include markers to confer selectable phenotypes, such as drug resistance, nutritional requirements, resistance to cytotoxic agents, or expression of surface proteins, for example, GFP (green fluorescent protein), puromycin, neomycin (Neo), hygromycin (Hyg), histidinol dehydrogenase gene (hisD) and guanine phosphosribosyltransferase (Gpt), and the like, and GFP (green fluorescent protein) and puromycin markers are preferably used.

Further, the present invention provides a pharmaceutical composition for preventing or treating atopic diseases, including: a composition that inhibits TSLP gene expression and includes porous silica particles carrying nucleic acid molecules that complementarily bind to at least a portion of TSLP mRNA.

Details of the nucleic acid molecules, porous silica particles, inhibition of TSLP gene expression, and the like are as described above.

The pharmaceutical composition of the present invention may exhibit effects of preventing or treating atopic diseases, wherein the above effects may be effects to be achieved by inhibiting the expression of TSLP gene with the nucleic acid molecules of the present invention.

Examples of atopic diseases that are diseases to be prevented or treated by the pharmaceutical composition of the present invention may include at least one selected from the group consisting of allergic diseases such as: bronchial asthma, allergic rhinitis, urticaria, atopic dermatitis, allergic conjunctivitis, allergic dermatitis, allergic contact dermatitis, inflammatory skin disease, pruritus or food allergy, but it is not particularly limited thereto. That is, the disease is not particularly limited as long as it corresponds to a disease due to overexpression of TSLP.

"Atopic dermatitis" means a condition in which the infected site of the skin is changed by the atopic dermatitis, and which includes both a condition considered as a skin disease and a condition substantially not regarded as a skin disease.

The pharmaceutical composition of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The pharmaceutical composition of the present invention is applicable in a form of any formulation containing the nucleic acid molecule of the present invention as an active ingredient, and may be prepared in oral or parenteral formulations. The pharmaceutical formulations of the present invention may include forms suitable for oral, rectal, nasal, topical (including the cheek and sublingual), subcutaneous, vaginal or parenteral (intramuscular, subcutaneous) administration. Alternatively, forms suitable for administration by inhalation or insufflations may also be included.

The pharmaceutical composition of the present invention is administered in a pharmaceutically effective amount. Effective dose levels may be determined depending on types of disease of the patient, severity, activity of drug, sensitivity to drug, administration time, administration route and rate of release, duration of treatment, factors including concurrent medications, and other factors well known in the medical field. The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the pharmaceutical composition according to the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the nucleic acid molecules of the present invention used. Generally, the amount may be calculated on the basis of EC50, which is generally determined to be effective in in *vivo* animal models and *in vitro,* for example, from 0.01 $\mu$g to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The pharmaceutical composition of the present invention may further include a compound to maintain/increase one or more of active ingredients exhibiting the same or similar functions in relation to treatment of fibroproliferative diseases or the solubility and/or absorption of at least one active ingredient. Further, the composition may also optionally include chemotherapeutic agents, anti-inflammatory agents, antiviral agents and/or immunomodulators and the like.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

Furthermore, the present invention provides a cosmetic composition for prevention or improvement of atopic diseases, including; a composition that inhibits TSLP gene expression and includes porous silica particles carrying nucleic acid molecules that complementarily bind to at least a portion of TSLP mRNA.

Specific details regarding the nucleic acid molecule, porous silica particles, inhibition of TSLP gene expression, atopic diseases, and the like are the same as described above.

The cosmetic composition of the present invention may exhibit effects of preventing or improving atopic diseases, wherein the above effects may be effects to be achieved by inhibiting the expression of TSLP gene with the nucleic acid molecules of the present invention.

The cosmetic composition of the present invention may further include components typically used in the cosmetic composition, and may include, for example, conventional auxiliaries such as antioxidants, stabilizers, solubilizers, vitamins, pigments and flavors, as well as carriers, but it is not particularly limited thereto.

Products to which the above composition can be added may include, for example, cosmetics such as astringent toilet water, soft toilet water, nourishing toilet water, various creams, essences, packs and foundations, cleansing agents, facial cleansers, soaps, treatments, cosmetic solutions, etc., but it is not particularly limited thereto.

Specific formulations of the cosmetic composition according to the present invention may include skin lition, skin softener, skin toner, astringent lotion, milk lotion, moisture lotion, nutrition lotion, massage cream, nutrition cream, moisture cream, hand cream, essence, nutrition essence, pack, soap, shampoo, cleansing foam, cleansing lotion, cleansing cream, body lotion, body cleanser, emulsion, lipstick, makeup base, foundation, press powder, loose powder, eye shadow, etc., but it is not particularly limited thereto.

The present invention also relates to a method for treatment of atopic diseases.

The method for treatment of atopic diseases according to the present invention may include administering porous silica particles that carry a substance for inhibiting TSLP expression to a subject.

Substances capable of inhibiting TSLP expression may be within the above-described range.

The porous silica particles may be within the above-exemplified range or may be prepared by any method/condition within the above-exemplified range.

The subject may be a mammal including a human, and specifically a human.

The substance for inhibiting TSLP expression may be formulated in a method within the above-described range in a form of the composition.

Administration methods are not limited and may include, for example, oral, rectal, nasal, topical (including buccal and sublingual), subcutaneous, vaginal or parenteral (including intramuscular, subcutaneous and intravenous) administration, or administration by inhalation or insufflation.

The present invention also relates to use of porous silica particles that carry a substance for inhibiting TSLP expression in the manufacture of a pharmaceutical composition for preventing or treating atopic diseases.

The substance for inhibiting TSLP expression may be within the above-described range.

The porous silica particles may belong to the above-exemplified range or may be prepared according to the methods/conditions within the above-exemplified range.

Hereinafter, the present invention will be described in detail with reference to the following examples.

Hereinafter, siRNA used in the present invention may be abbreviated as 'siTSLP'. Likewise, porous silica particles of the present invention may be abbreviated as 'DegradaBALL or DDV', and DegradaBALL carrying siTSLP may be abbreviated as 'LEM-siTSLP'.

**Experimental Procedure**

**1. Experimental materials**

DegradaBALL and TAMRA-combined DegradaBALL were provided by Lemonex, Inc. (Seoul, Korea), and cell counting kit-8 was purchased from Dojindo molecular technologies, Inc. (Maryland, USA). TGF-β was purchased from Peprotech (New Jersey, USA), and 10% phosphate buffered saline (PBS), Dulbecco's Modified Eagle's Medium (DMEM), fetal bovine serum (FBS), Roswell Park Memorial Laboratory 1640 (RPMI 1640), penicillin-streptomycin and 0.05% trypsin-EDTA were purchased from WelGene (Korea). All nucleic acid molecules were synthesized by Lemonex (Seoul, Korea), and their sequences and nucleic acid molecule sequences used throughout the present specification are shown in Table 1 below. All PCR primers were purchased from Cosmogenetech (Seoul, Korea). Anti-mouse TSLP antibodies were purchased from Abcam (Cambridge, UK) and anti-mouse collagen 1 and 3 antibodies were purchased from Invitrogen (Carlsbad, CA, USA). Trizol cell lysis solution was purchased from Molecular Probes Invitrogen (Carlsbad, CA, USA) and all PCR reagents were purchased from TaKaRa Bio Inc. (Shiga, Japan). All chemicals were used as received.

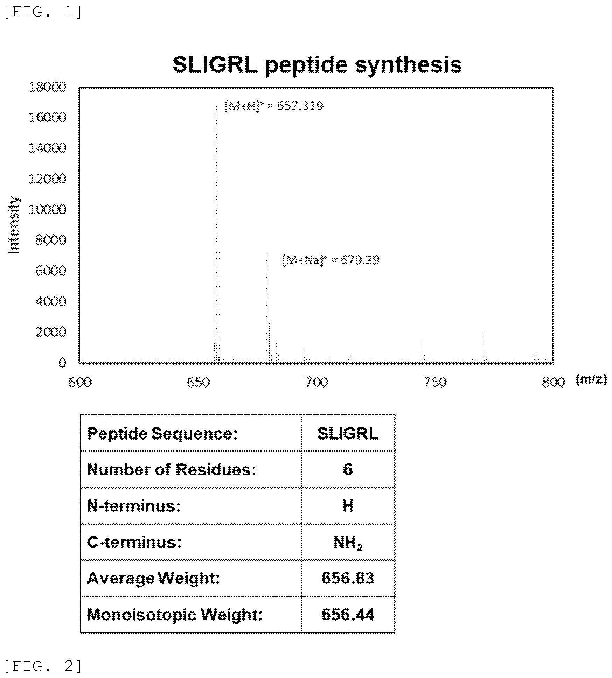

SLIGRL peptide used to induce TSLP expression was synthesized by Lemonex (Seoul, South Korea), MS analysis results of the synthesized peptide are shown in FIG. 1.

Nucleotide sequences can be searched in the US National Center for Biological Information (http://www.ncbi.nim.nih.gov) and are listed as thymic stromal lymphopoietin in the full official name with the official symbol of TSLP. Further, human sequences under approval number NM_033035.4 were used to design siRNA, dsRNA, and antisense RNA sequences for human TSLP. The siRNA was designed thorugh Dharmacon RNAi & Gene Expression service from GE Healthcare (http://dharmacon.gelifesciences.com/designcenter/?redirect =true) among internet sites to provide siRNA designs. In this regard, 10 (ten) sequences from siRNA SEQ ID NOs: 1-10, wherein a GC content of the target sequence ranges from 30% to 70%, were selected among the designed DNA fragments. Further, 13 sequences from SEQ ID NOs: 11 - 23 were designed as siRNA sequences with GC contents of 30% to 70% by the inventor. The finally selected 23 siRNAs were produced by the order from Bioneer (http://www.bioneer.co.kr). In this regard, a sense sequence and an antisense sequence for the target sequence of TSLP were designed for siRNA production, respectively. In addition, siRNA and dsRNA were prepared by specific base pair binding of each single sequence, that is, the sense sequence and the antisense sequence.

Specific information of the afore-mentioned sequences is concretely indicated in the attached sequence list and Table 2 below.

[TABLE 2]

| | |
|---|---|
| Target sequence 1: SEQ ID NO: 126 5'-GCAGCCUAUCUCAGUACUA-3' (Position in gene sequence: 140) | Sense strand: SEQ ID NO: 70 5'-GCAGCCUAUCUCAGUACUAUU-3' |
| | Antisense strand: SEQ ID NO: 93 5'-UAGUACUGAGAUAGGCUGCUU-3' |
| | dsRNA: SEQ ID NO: 24 5'-GCAGCCUAUCUCAGUACUAUUUCUA-3' |
| Target sequence 2: SEQ ID NO: 127 5'-GCCUAUCUCAGUACUAUUU-3' (Position in gene sequence: 143) | Sense strand: SEQ ID NO: 71 5'-GCCUAUCUCAGUACUAUUUUU-3' |
| | Antisense strand: SEQ ID NO: 94 5'-AAAUAGUACUGAGAUAGGCUU-3' |
| | dsRNA: SEQ ID NO: 25 5'-GCCUAUCUCAGUACUAUUUCUAAA G-3' |
| Target sequence 3: SEQ ID NO: 128 5'-GCCACAUUGCCUUACUGAA-3' (Position in gene sequence: 235) | Sense strand: SEQ ID NO: 72 5'-GCCACAUUGCCUUACUGAAUU-3' |
| | Antisense strand: SEQ ID NO: 95 5'-UUCAGUAAGGCAAUGUGGCUU-3' |
| | dsRNA: SEQ ID NO: 26 5'-GCCACAUUGCCUUACUGAAAUCCA G-3' |
| Target sequence 4: SEQ ID NO: 129 5'-CCACAUUGCCUUACUGAAA-3' (Position in gene sequence: 236) | Sense strand: SEQ ID NO: 73 5'-CCACAUUGCCUUACUGAAAUU-3' |
| | Antisense strand: SEQ ID NO: 96 5'-UUUCAGUAAGGCAAUGUGGUU-3' |
| | dsRNA: SEQ ID NO: 27 5'-CCACAUUGCCUUACUGAAAUCCAGA-3' |
| Target sequence 5: SEQ ID NO: 130 5'-UCCAGAGCCUAACCUUCAA-3' (Position in gene sequence: 255) | Sense strand: SEQ ID NO: 74 5'-UCCAGAGCCUAACCUUCAAUU-3' |
| | Antisense strand: SEQ ID NO: 97 5'-UUGAAGGUUAGGCUCUGGAUU-3' |
| | dsRNA: SEQ ID NO: 28 5'-UCCAGAGCCUAACCUUCAAUCCCCA-3' |
| Target sequence 6: SEQ ID NO: 131 5'-CCAGAGCCUAACCUUCAAU-3' (Position in gene sequence: 256) | Sense strand: SEQ ID NO: 75 5'-CCAGAGCCUAACCUUCAAUUU-3' |
| | Antisense strand: SEQ ID NO: 98 5'-AUUGAAGGUUAGGCUCUGGUU-3' |
| | dsRNA: SEQ ID NO: 29 5'-CCAGAGCCUAACCUUCAAUCCCACC-3' |

(continued)

| | |
|---|---|
| Target sequence 7: SEQ ID NO: 132 5'-GCGUCGCUCGCCAAAGAAA-3' (Position in gene sequence: 290) | Sense strand: SEQ ID NO: 76 5'-GCGUCGCUCGCCAAAGAAAUU-3' |
| | Antisense strand: SEQ ID NO: 99 5'-UUUCUUUGGCGAGCGACGCUU-3' |
| | dsRNA: SEQ ID NO: 30 5'-GCGUCGCUCGCCAAAGAAAUGUUCG-3' |
| Target sequence 8: SEQ ID NO: 133 5'-CCAAAGAAAUGUUCGCCAU-3' (Position in gene sequence: 300) | Sense strand: SEQ ID NO: 77 5'-CCAAAGAAAUGUUCGCCAUUU-3' |
| | Antisense strand: SEQ ID NO: 100 5'-AUGGCGAACAUUUCUUUGGUU-3' |
| | dsRNA: SEQ ID NO: 31 5'-CCAAAGAAAUGUUCGCCAUGAAAAC-3' |
| Target sequence 9: SEQ ID NO: 134 5'-GCUUCAAUCGACCUUUACU-3' (Position in gene sequence: 468) | Sense strand: SEQ ID NO: 78 5'-GCUUCAAUCGACCUUUACUUU-3' |
| | Antisense strand: SEQ ID NO: 101 5'-AGUAAAGGUCGAUUGAAGCUU-3' |
| | dsRNA: SEQ ID NO: 32 5'-GCUUCAAUCGACCUUUACUGAAACA-3' |
| Target sequence 10: SEQ ID NO: 135 5'-UCAAUCGACCUUUACUGAA-3' (Position in gene sequence: 471) | Sense strand: SEQ ID NO: 79 5'-UCAAUCGACCUUUACUGAAUU-3' |
| | Antisense strand: SEQ ID NO: 102 5'-UUCAGUAAAGGUCGAUUGAUU-3' |
| | dsRNA: SEQ ID NO: 33 5'-UCAAUCGACCUUUACUGAAACAACA-3' |
| Target sequence 11: SEQ ID NO: 136 5'-GCCUUACUAUAUGUUCUGUC-3' (Position in gene sequence: 32) | Sense strand: SEQ ID NO: 80 5'-GCCUUACUAUAUGUUCUGUCUU-3' |
| | Antisense strand: SEQ ID NO: 103 5'-GACAGAACAUAUAGUAAGGCUU-3' |
| | dsRNA: SEQ ID NO: 34 5'-GCCUUACUAUAUGUUCUGUCAGUUU-3' |
| Target sequence 12: SEQ ID NO: 137 5'-CCUUACUAUAUGUUCUGUCAG-3' (Position in gene sequence: 33) | Sense strand: SEQ ID NO: 81 5' -CCUUACUAUAUGUUCUGUCAGUU-3' |
| | Antisense strand: SEQ ID NO: 104 5'-CUGACAGAACAUAUAGUAAGGUU-3' |
| | dsRNA: SEQ ID NO: 35 5'-CCUUACUAUAUGUUCUGUCAGUUUC-3' |
| Target sequence 13: SEQ ID NO: 138 5'-CAGGAAAAUCUUCAUCUUAC-3' | Sense strand: SEQ ID NO: 82 5'-CAGGAAAAUCUUCAUCUUACUU-3' |
| | Antisense strand: SEQ ID NO: 105 |
| (Position in gene sequence: 61) | 5'-GUAAGAUGAAGAUUUUCCUGUU-3' |
| | dsRNA: SEQ ID NO: 36 5'-CAGGAAAAUCUUCAUCUUACAACUU-3' |

(continued)

| Target sequence 14: SEQ ID NO: 139 5'-GCUGGUGUUAACUUACGACU-3' (Position in gene sequence: 91) | Sense strand: SEQ ID NO: 83 5'-GCUGGUGUUAACUUACGACUUU-3' |
|---|---|
| | Antisense strand: SEQ ID NO: 106 5'-AGUCGUAAGUUAACACCAGCUU-3' |
| | dsRNA: SEQ ID NO: 37 5'-GCUGGUGUUAACUUACGACUCUUCA-3' |
| Target sequence 15: SEQ ID NO: 140 5'-GGUGUUAACUUACGACUUCA-3' (Position in gene sequence: 94) | Sense strand: SEQ ID NO: 84 5'-GGUGUUAACUUACGACUUCAUU-3' |
| | Antisense strand: SEQ ID NO: 107 5'-UGAAGUCGUAAGUUAACACCUU-3' |
| | dsRNA: SEQ ID NO: 38 5'-GGUGUUAACUUACGACUUCACUAAC-3' |
| Target sequence 16: SEQ ID NO: 141 5'-CACUAACUGUGACUUUGAG-3' (Position in gene sequence: 112) | Sense strand: SEQ ID NO: 85 5'-CACUAACUGUGACUUUGAGUU-3' |
| | Antisense strand: SEQ ID NO: 108 5'-CUCAAAGUCACAGUUAGUGUU-3' |
| | dsRNA: SEQ ID NO: 39 5'-CACUAACUGUGACUUUGAGAAGAUU-3' |
| Target sequence 17: SEQ ID NO: 142 5'-GACCUGAUUACAUAUAUGAG-3' (Position in gene sequence: 167) | Sense strand: SEQ ID NO: 86 5'-GACCUGAUUACAUAUAUGAGUU-3' |
| | Antisense strand: SEQ ID NO: 109 5'-CUCAUAUAUGUAAUCAGGUCUU-3' |
| | dsRNA: SEQ ID NO: 40 5'-GACCUGAUUACAUAUAUGAGUGGGA-3' |
| Target sequence 18: SEQ ID NO: 143 5'-CCGAGUUCAACAACACCGU-3' (Position in gene sequence: 201) | Sense strand: SEQ ID NO: 87 5'-CCGAGUUCAACAACACCGUUU-3' |
| | Antisense strand: SEQ ID NO: 110 5'-ACGGUGUUGUUGAACUCGGUU-3' |
| | dsRNA: SEQ ID NO: 41 5'-CCGAGUUCAACAACACCGUCUCUUG-3' |
| Target sequence 19: SEQ ID NO: 144 5'-ACCGUCUCUUGUAGCAAUCG-3' (Position in gene sequence: 215) | Sense strand: SEQ ID NO: 88 5'-ACCGUCUCUUGUAGCAAUCGUU-3' |
| | Antisense strand: SEQ ID NO: 111 5'-CGAUUGCUACAAGAGACGGUUU-3' |
| | dsRNA: SEQ ID NO: 42 5'-ACCGUCUCUUGUAGCAAUCGGCCAC-3' |

(continued)

| | |
|---|---|
| | Sense strand: SEQ ID NO: 89 5'-AAGGCUGCCUUAGCUAUCUGUU-3' |
| Target sequence 20: SEQ ID NO: 145 5'-AAGGCUGCCUUAGCUAUCUG-3' (Position in gene sequence: 326) | Antisense strand: SEQ ID NO: 112 5'-CAGAUAGCUAAGGCAGCCUUUU-3' |
| | dsRNA: SEQ ID NO: 43 5'-AAGGCUGCCUUAGCUAUCUGGUGCC-3' |
| | Sense strand: SEQ ID NO: 90 5'-CGGAAACUCAGAUAAAUGCUU -3' |
| Target sequence 21: SEQ ID NO: 146 5'-CGGAAACUCAGAUAAAUGC-3' (Position in gene sequence: 360) | Antisense strand: SEQ ID NO: 113 5'-GCAUUUAUCUGAGUUUCCGUU-3' |
| | dsRNA: SEQ ID NO: 44 5'-CGGAAACUCAGAUAAAUGCUACUCA-3' |
| | Sense strand: SEQ ID NO: 91 5'-CCAAUAAAUGUCUGGAACAAUU-3' |
| Target sequence 22: SEQ ID NO: 147 5'-CCAATAAATGTCTGGAACAA-3' (Position in gene sequence: 420) | Antisense strand: SEQ ID NO: 114 5'-UUGUUCCAGACAUUUAUUGGUU-3' |
| | dsRNA: SEQ ID NO: 45 5'-CCAATAAATGTCTGGAACAAGUGUC-3' |
| | Sense strand: SEQ ID NO: 92 5'-CAAGGAUUGUGGCGUCGCUUU-3' |
| Target sequence 23: SEQ ID NO: 148 5'-CAAGGAUUGUGGCGUCGCU-3' (Position in gene sequence: 442) | Antisense strand: SEQ ID NO: 115 5'-AGCGACGCCACAAUCCUUGUU-3' |
| | dsRNA: SEQ ID NO: 46 5'-CAAGGAUUGUGGCGUCGCUGCUUCA-3' |

## 2. Porous silica particles (DDV or DegradaBALL)

### 2-1. Preparation of porous silica particles

(1) Preparation of porous silica particles

1) Preparation of small pore particles

960 mL of distilled water (DW) and 810 mL of MeOH were put into a 2 L round bottom flask. 7.88 g of CTAB was added to the flask, followed by rapid addition of 4.52 mL of 1 M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8000 rpm and 25 °C for 10 minutes to remove the supernatant, centrifuged at 8000 rpm and 25 °C for 10 minutes, and washed five times with ethanol and distilled water alternately.

Thereafter, the resultant product was dried in an oven at 70 °C to harvest 1.5 g of powdery microporous silica particles (pore average diameter of 2 nm and particle size of 200 nm).

2) Pore expansion

1.5 g of microporous silica particle powder was added to 10 ml of ethanol and subjected to ultrasonic dispersion, and 10 ml of water and 10 ml of TMB (trimethyl benzene) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8000 rpm for 10 minutes at 25 °C to remove the supernatant, and centrifuged at 8000 rpm for 10 minutes at 25 °C and washed five times with ethanol and distilled water alternately.

Then, the product was dried in an oven at 70 °C to harvest powdery porous silica particles (pore diameter of 10 to 15 nm, and particle size of 200 nm).

3) Calcination

The porous silica particles prepared in 2) were put in a glass vial, heated at 550 °C for 5 hours, and cooled slowly to room temperature after completing the reaction to prepare particles.

(2) Preparation of porous silica particles

Porous silica particles were prepared by the same method as Example 2-1-(1), except that the reaction conditions at the time of pore expansion were changed to 140 °C and 72 hours.

(3) Preparation of porous silica particles (10 L scale)

Porous silica particles were prepared by the same method as Example 2-1-(1), except that a 5 times larger container was used and each material was used in a 5 times capacity.

(4) Preparation of porous silica particles (particle size of 300 nm)

Porous silica particles were prepared by the same method as Example 2-1-(1), except that 920 ml of distilled water and 850 ml of methanol were used to prepare the small pore particles.

(5) Preparation of porous silica particles (particle size of 500 nm)

Porous silica particles were prepared by the same method as Example 2-1-(1), except that 800 ml of distilled water, 1010 ml of methanol, and 10.6 g of CTAB were used to prepare the small pore particles.

(6) Preparation of porous silica particles (particle size of 1000 nm)

Porous silica particles were prepared by the same method as Example 2-1-(1), except that 620 ml of distilled water, 1380 ml of methanol, and 7.88 g of CTAB were used to prepare the small pore particles.

(7) Preparation of porous silica particles (pore diameter of 4 nm)

Porous silica particles were prepared by the same method as Example 2-1-(1), except that 2.5 mL of TMB was used for pore expansion.

(8) Preparation of porous silica particles (pore diameter of 7 nm)

Porous silica particles were prepared by the same method as Example 2-1-(1), except that 4.5 mL of TMB was used for pore expansion.

(9) Preparation of porous silica particles (pore diameter of 17 nm)

Porous silica particles were prepared by the same method as Example 2-1-(1), except that 11 mL of TMB was used for pore expansion.

(10) Preparation of porous silica particles (pore diameter of 23 nm)

Porous silica particles were prepared by the same method as Example 2-1-(1), except that 12.5 mL of TMB was used for pore expansion.

(11) Preparation of porous silica particles (dual modification)

1) Preparation of small pore particles

Small pore particles were prepared by the same method as Example 2-1- (1) -1).

2) Pore expansion

Small pore particles were reacted with TMB, cooled and centrifuged by the same method as Example 2-1-(1)-2) to remove the supernatant. Thereafter, the remaining solution was centrifuged under the same conditions as Example 2-1-(1) -2), washed three times with ethanol and distilled water alternately, and then dried under the same conditions as Example 2-1-(1)-2), thereby harvesting powdery porous silica particles (pore diameter 10 to 15 nm, and particle size of 200 nm).

3) Surface modification

After dispersing 0.8 g to 1 g of porous silica particles having expanded pores in 50 mL of toluene, 5 mL of (3-aminopropyl)triethoxysilane was added thereto, followed by heating under reflux at 120 °C for 12 hours. The procedure is followed by the washing and drying procedures described above, followed by 1 mL of triethylene glycol (PEG3, 2-[2-(2-methoxyethoxy)ethoxy] acetic acid) and 100 mg of EDC (1-ethyl-3-(3-dimethylaminopropyl)carbodiimide) and 200 mg of N-hydroxysuccinimide (NHS) were dispersed in 30 mL of PBS and allowed to react at room temperature for 12 hours under stirring. The product was then washed and dried.
Since the reaction solution of the previous step remained inside of the pores, the inside of the pores was not modified.

4) Washing inside pores

800 mg of surface-modified particle powder was dissolved in 40 ml of 2M HCl/ethanol and refluxed under vigorous stirring for 12 hours.
Thereafter, the cooled reaction solution was centrifuged at 8000 rpm for 10 minutes to remove the supernatant, centrifuged at 8000 rpm and 25 °C for 10 minutes, and washed five times with ethanol and distilled water alternately.
Thereafter, the product was dried in an oven at 70 °C, thereby harvesting powdery porous silica particles.

5) Modifying inside pores

① A propyl group was introduced into the pore in the same manner as the method of Example 2-2-(2)-1) described below.

② An octyl group was introduced into the pore in the same manner as the method of Example 2-2-(2)-2) described below.

**2-2. Surface modification of porous silica particles**

(1) Positively charging

1) Particles with particle size of 300 nm

The porous silica particles of Example 2-1-(4) were reacted with (3-aminopropyl)triethoxysilane (APTES) to be positively charged.
Specifically, 100 mg of porous silica particles were dispersed in a 10 mL toluene in a 100 mL round bottom flask with a bath sonicator. Then, 1 mL of APTES was added and stirred at 400 rpm and 130 °C for 12 hours.
After the reaction, the product was slowly cooled to room temperature and centrifuged at 8000 rpm for 10 minutes to remove the supernatant, further centrifuged at 8000 rpm and 25 °C for 10 minutes, and then washed five times with ethanol and distilled water alternately.
Thereafter, the product was dried in an oven at 70 °C to harvest powdery porous silica particles having an amino group on the surface thereof and inside of the pores.

2) Particles with particle size of 200 nm

① The porous silica particles of Example 2-1-(1) were positively charged by reacting the particles with (3-aminopropyl)triethoxysilane (APTES), and were modified in the same manner as the method of 2-2-(1)-1, except that 0.4 ml of APTES was added and the reaction time was 3 hours.

② The porous silica particles of Example 2-1-(9) were positively charged by reacting the particles with (3-aminopropyl)triethoxysilane (APTES), and were modified in the same manner as the method of 2-2-(1)-1.

③ The porous silica particles of Example 2-1-(10) were positively charged by reacting the particles with (3-aminopropyl)triethoxysilane (APTES), and were modified in the same manner as the method of 2-2-(1)-1.

(2) Introduction of hydrophobic groups

1) Propyl group

The porous silica particles of Example 2-1-(1) were reacted with trimethoxy(propyl)silane to introduce propyl groups into the surface of the particles and inside of the pores, and were subjected to modification by the same method as Example 2-2-(1), except that 0.35 ml of trimethoxy(propyl)silane was added instead of APTES, followed by 12 hours of reaction.

2) Octyl group

The porous silica particles of Example 2-1-(1) were reacted with trimethoxy-n-octylsilane to introduce octyl groups on the surface of the particles and inside of the pores, and were subjected to modification by the same method as Example 2-2-(1), except that 0.5 ml of trimethoxy-n-octylsilane was added instead of APTES, followed by 12 hours of reaction.

(3) Negatively charging

1) Carboxyl group

The porous silica particles of Example 2-1-(1) were negatively charged by reacting the particles with succinic anhydride.

Further, the charged particles were subjected to modification in the same manner as the method of Example 2-2-(1)-1), except that DMSO (dimethyl sulfoxide) was used instead of toluene, 80 mg of succinic anhydride was added instead of APTES to allow reaction at room temperature for 24 hours under stirring, and DMSO was used instead of distilled water.

2) Thiol group

The particles were subjected to modification in the same manner as the method of Example 2-2-(1)-1), except that 1.1 mL of MPTES was used instead of APTES.

3) Sulfonic acid group

100 mg of the porous silica nanoparticles of Example 2-2-(3)-2) were dispersed in 1 mL of 1 M aqueous sulfuric acid solution and 20 mL of 30% hydrogen peroxide solution, and stirred at room temperature to induce oxidation, thereby oxidizing a thiol group into a sulfonic acid group. Thereafter, the product was washed and dried in the same manner as the method of Example 2-2-(1)-1).

## 3. Support of nucleic acid molecules

10 µg of porous silica particles of Example 2-2-(1)-2)-② and 50 pmol of nucleic acid molecules were mixed under 1 × PBS conditions, and then placed at room temperature for 30 minutes to complete loading.

The nucleic acid molecules, that is: siRNA composed of a sense RNA having the sequence of SEQ ID NO: 70 and an antisense RNA having the sequence of SEQ ID NO: 93 (hereinafter, siTSLP #1); siRNA composed of a sense RNA having the sequence of SEQ ID NO: 83 and an antisense RNA having the sequence of SEQ ID NO: 106 (hereinafter siTSLP #14); and/or siRNA composed of a sense RNA having the sequence of SEQ ID NO: 90 and an antisense RNA having the sequence of SEQ ID NO: 113 (hereinafter siTSLP #21) were used to implement the following experiment. However, in the following description, if there is a further description of sequences constituting the nucleic acid molecule (e.g. sense RNA, antisense RNA, dsRNA), it can be understood that such nucleic acid molecules having the separately specified sequences were loaded or contained.

Hereinafter, the DDV carrying siTSLP#1 is expressed as LEM-siTSLP#1, the DDV carrying siTSLP#14 is expressed as LEM-siTSLP#14, and the DDV carrying siTSLP#21 is expressed as LEM-siTSLP#21.

### 4. Determination of cell viability

A549 and HaCaT cells were seeded in 96-well culture plates with 100 $\mu$l of growth medium (50-70% confluency) at a density of 10,000 cells per well. Cells were treated with an appropriate concentration of DegradaBALL (Example 2-2-(1)-2)-②) in a serum-containing medium and incubated at 37 °C for 24 hours. After incubation, the cells were washed twice with 1 x PBS, and then 100 $\mu$l of serum-free medium containing 10 $\mu$l of CCK-8 was added, followed by further incubation for 1 hour. The optical density of each well in the culture plate was measured at 450 nm wavelength. Mean and standard deviation of the deviation of triplicates were calculated and plotted.

### 5. Cell-based TSLP knockdown assay

### 5-1. Culture of human skin Keratinocyte cell-line HaCaT cells

In order to investigate whether human TSLP-specific siRNA, dsRNA and antisense RNA oligonucleotides can inhibit the production of TSLP in skin cells, keratinocyte HaCaT cell-line (CLC cell-line service, Germany) was used. HaCaT cell-lines were incubated in DMEM culture (Gibco BRL, USA) containing 10% fetal bovine serum (FBS; Gibco BRL, USA) and antibiotics. The culture dishes used herein were 100 mm culture dish, 6-well plate and 24-well plate, and the incubation was conducted in a 37 °C incubator with 5% $CO_2$. The cultures were exchanged every two days and subcultured just before the cells were excessively proliferated.

### 5-2. LEM-siTSLP treatment on HaCaT cells

LEM-siTSLP (25 pmol) dispersed in a serum-free medium was used to treat HaCaT cells cultured in 24-well plate. After incubation in 5% $CO_2$ incubator at 37 °C for 6 hours, the serum-free culture was removed and washed twice with 1 × PBS, followed by replacing the medium with a serum-containing cell medium. After 6 hours, the serum-containing culture medium was again removed and washed with 1 x PBS. The cells were treated with SLIGRL peptide (200 $\mu$M) in the serum- containing medium. Then, in order to confirm induction of TSLP, total RNA was extracted using Trizol (Invitrogen, USA) after 0, 6, 12 and 24 hours of culture.

Further, in order to measure the duration of TSLP knockdown by LEM-siTSLP, PAM212 cells were treated with LNP-siTSLP (mouse) (25 pmol siTSLP) containing LNP and siTSLP (mouse) (25 pmol), respectively, in a serum-free medium. After incubation in a 5% $CO_2$ incubator at 37 °C for 6 hours, the serum-free culture was removed and washed twice with 1 × PBS, followed by replacing the medium with a serum-containing cell medium. After incubation for the indicated times, the cells were treated with SLIGRL (200 $\mu$M) in the serum-containing culture medium. Then, the cells were further incubated for 12 hours for TSLP induction. Total RNA was extracted using Trizol.

### 6. RT-PCR

1$\mu$g of total RNA and nuclease-free water were mixed to prepare 16 $\mu$L of mixture, which in turn was reacted at 70 °C for 5 minutes to denature RNA. After cooling rapidly on ice, the evaporated solution was collected through short centrifugation. Then, 4 $\mu$L of Reverse Transcription Master Premix (Elpis Biotech, contain random hexamer, 5x ready-to-use mix, cat # EBT-1511) was added, mixed well and reacted at 42 °C for 1 hour. Subsequently, after reacting for 5 minutes at 94 °C, the product was cooled on ice and then stored at -20 °C.

Following then, 7.4 $\mu$L of nuclease-free water, 0.8 $\mu$L of each of the forward and reverse primers (5 $\mu$M), 1 $\mu$L of the cDNA template synthesized above, and 10 $\mu$L of Power SYBR™ Green PCR Master Mix (appliedbiosystems, 2x ready-to-use mix, cat # 4367659) were mixed to prepare a mixed solution.

RT-PCR primer sequences used for mRNA expression analysis are shown in Table 3 below.

[TABLE 3]

| mRNA type | Forward primer | Reverse primer |
|---|---|---|
| hTSLP (Human TSLP) | GAGCCGCAGGCACCCTCTCA (SEQ ID NO: 116) | GCCCCAACTAACCCTCAGGGAGT (SEQ ID NO: 117) |
| mTSLP (Mouse TSLP) | GCAAGCCAGCTTGTCTCCTGA (SEQ ID NO: 118) | GGCAGTGGTCATTGAGGGCTT (SEQ ID NO: 119) |
| hGAPDH (Human GAPDH) | TCACTGCCACCCAGAAGACTG (SEQ ID NO: 120) | GGATGACCTTGCCCACAGC (SEQ ID NO: 121) |
| mGAPDH (Mouse GAPDH) | TGACCTCAACTACATGGTCTACA (SEQ ID NO: 122) | CTTCCCATTCTCGGCCTTG (SEQ ID NO: 123) |

After denaturation at 95 °C for 3 minutes, 2-step PCR cycles were repeated at 95 °C for 10 seconds and at 60 °C for 5 seconds (GAPDH: 30 cycles, TSLP: 36 cycles). The final reaction product was placed on on 1% agarose gel for confirmation.

**7. Biological tissue imaging at administered site of siTSLP and porous silica particles**

20 $\mu$l of LEM-siTSLP (0.7 nmol siTSLP (mouse), 150 $\mu$g DDV) (FITC-conjugated siTSLP (mouse) and TAMRA-DegradaBALL) was injected into the mouse buccal skin. The DDV used herein was the porous silica particles of Example 2-2-(1)-2)-②, and the siTSLP (mouse) used herein was a combination of the sense RNA sequence (5'-CGAGCAAAUCGAGGACUGUdTdT-3' (SEQ ID NO: 124)) and the antisense RNA sequence (5'-ACAGUCCUCGAUUUGCUCGdTdT-3' (SEQ ID NO: 125)). After the sacrifice of the mouse, fluorescent images of the excised mouse buccal skin were taken using a FOBI imaging device (NeoScience Co., Ltd., Seoul, Korea). The obtained skin sample was placed in 4% PFA solution. The sample was inserted into paraffin and cut to 10 $\mu$m thickness. After dehydration, the sample section was stained with DAPI. The sample was observed under a BX71 microscope equipped with a 20 x objective lens (Olympus, Tokyo, Japan).

**Experimental result**

**1. Analysis of TSLP expression inhibition by nucleic acid molecules of the present invention**

In order to determine TSLP expression inhibition rate of the nucleic acid molecules (siRNA or dsRNA) prepared using the sequences listed in the above Table 2, the nucleic acid molecules were transfected into HaCaT cells using Lipofectamine 2000 (Invitrogen, USA) and cationic liposomes, followed by determining TSLP expression inhibition efficiency.

TSLP expression inhibition rates when using the nucleic acid molecules (siRNA or dsRNA) are shown in Table 4 below.

[TABLE 4]

| SEQ ID NO. ("/" means pair between sense strand and antisense strand) | Expression inhibition rate (%) | SEQ ID NO. | Expression inhibition rate (%) |
|---|---|---|---|
| 70/93 | 88.18 | 82/105 | 95.88 |
| 24 | 92.91 | 36 | 67.53 |
| 71/94 | 93.57 | 83/106 | 95.72 |
| 25 | 98.77 | 37 | 87.07 |
| 72/95 | 95.29 | 84/107 | 93.79 |
| 26 | 87.14 | 38 | 79.14 |

(continued)

| SEQ ID NO. ("/" means pair between sense strand andantisense strand) | Expression inhibition rate (%) | SEQ ID NO. | Expression inhibition rate (%) |
|---|---|---|---|
| 73/96 | 74.17 | 85/108 | 96.22 |
| 27 | 97.32 | 39 | 94.87 |
| 74/97 | 94.59 | 86/109 | 93.17 |
| 28 | 96.18 | 40 | 74.35 |
| 75/98 | 88.74 | 87/110 | 88.90 |
| 29 | 97.07 | 41 | 83.50 |
| 76/99 | 94.36 | 88/111 | 92.48 |
| 30 | 88.11 | 42 | 42.11 |
| 77/100 | 89.57 | 89/112 | 64.82 |
| 31 | 98.42 | 43 | 27.08 |
| 78/101 | 93.28 | 90/113 | 79.64 |
| 32 | 84.57 | 44 | 93.58 |
| 79/102 | 88.91 | 91/114 | 95.19 |
| 33 | 59.03 | 45 | 68.92 |
| 80/103 | 67.23 | 92/115 | 78.94 |
| 34 | 81.29 | 46 | 74.38 |
| 81/104 | 96.73 | - | - |
| 35 | 93.44 | - | - |

### 2. Porous silica particles (DDV or DegradaBALL)

### 2-1. Identification of particle formation and pore expansion

Small pore particles and porous silica particles prepared in Experimental Examples 2-1-(1) to (3) were observed under a microscope to determine whether the small pore particles were uniformly formed or the pores were sufficiently expanded to uniformly form the porous silica particles (FIGS. 2 to 5).

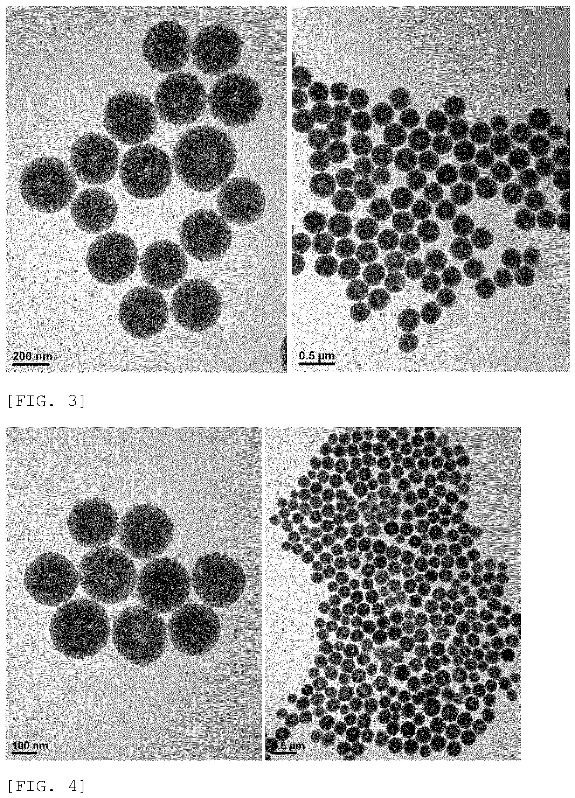

FIG. 2 is photographs of the porous silica particles in Experimental Example 2-1-(1), and FIG. 3 is photographs of the porous silica particles in Experimental Example 2-1-(2), and from these drawings, it can be seen that spherical porous silica particles having sufficiently expanded pores were formed evenly.

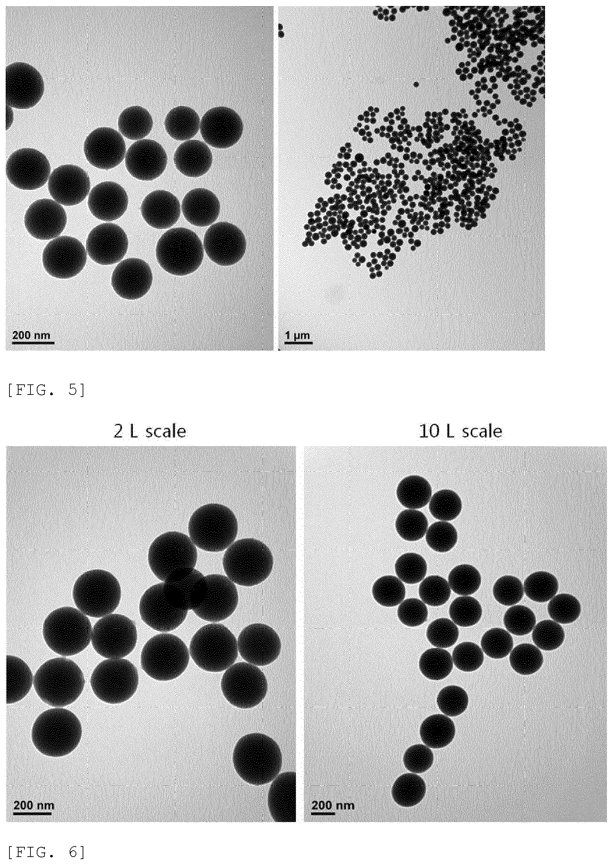

FIG. 4 is photographs of the small pore particles in Experimental Example 2-1-(1), and FIG. 5 is a comparative photograph of the small pore particles in Experimental Examples 2-1-(1) and 2-1-(3), and from these drawings, it can be seen that spherical small pore particles were formed evenly.

### 2-2. Calculation of BET surface area and pore volume

The surface area and pore volume of the small pore particles in Experimental Example 2-1-(1) and the porous silica particles of Experimental Examples 2-1-(1), (7), (8) and (10) were calculated. The surface area was calculated by Brunauer-Emmett-Teller (BET) method, and the pore size distribution was calculated by Barrett-Joyner-Halenda (BJH) method.

Micrographs of the particles are shown in FIG. 6, and the calculation results are shown in Table 5 below.

[TABLE 5]

| Section | Pore diameter (nm) | BET surface area ($m^2/g$) | Pore volume (mL/g) |
|---|---|---|---|
| Small pore particle in Experimental Example 2-1-(1) | 2.1 | 1337 | 0.69 |
| Experimental Example 2-1-(7) | 4.3 | 630 | 0.72 |
| Experimental Example 2-1-(8) | 6.9 | 521 | 0.79 |
| Experimental Example 2-1-(1) | 10.4 | 486 | 0.82 |
| Experimental Example 2-1-(10) | 23 | 395 | 0.97 |

**2-3. Identification of biodegradability**

In order to identify biodegradability of the porous silica particles in Experimental Example 2-1-(1), biodegradability at 37 °C in SBF (pH 7.4) was observed under a microscope at 0 hours, 120 hours and 360 hours, and results thereof are shown in FIG. 7.

Referring to FIG. 7, it can be seen that the porous silica particles are biodegraded and almost degraded after 360 hours.

**2-4. Measurement of absorbance ratio**

Absorbance ratio over time was measured according to Equation 1 below.

$$[Equation\ 1]$$

$$A_t/A_0$$

(wherein $A_0$ is absorbance of the porous silica particles measured by putting 5 ml of suspension containing 1 mg/ml of the porous silica particles into a cylindrical permeable membrane having pores with a pore diameter of 50 kDa, 15 ml of the same solvent as the suspension comes into contact with an outside of the permeable membrane, and the inside/outside of the permeable membrane are horizontally stirred at 60 rpm and 37 °C, and

$A_t$ indicates absorbance of the porous silica particles measured after lapse of "t" hours since $A_0$ was measured).

Specifically, 5 mg of porous silica particle powder was dissolved in 5 ml of SBF (pH 7.4). Thereafter, 5 ml of porous silica particle solution was placed in a permeable membrane having pores with a pore diameter of 50 kDa shown in FIG. 8. 15 ml of SBF was added to the outer membrane, and the SBF on the outer membrane was replaced every 12 hours. Degradation of the porous silica particles was performed at 37 °C under horizontal stirring at 60 rpm.

Then, the absorbance was measured by UV-vis spectroscopy and analyzed at $\lambda$ = 640 nm.

(1) Measurement of absorbance ratio

Absorbance ratio of the porous silica particles in Experimental Example 2-1-(1) was measured according to the above method, and results thereof are shown in FIG. 9.

Referring to FIG. 9, it can be seen that t, at which the absorbance ratio becomes 1/2, is about 58 hours to demonstrate very slow degradation.

(2) Particle size

Absorbances of the porous silica particles in Experimental Examples 2-1-(1), (5) and (6) were measured according to Equation 1 above, and results thereof are shown in FIG. 10 (SBF used as the suspension and the solvent).

Referring to FIG. 10, it can be seen that t is decreased as the particle size is increased.

(3) Average pore diameter

Absorbances of the porous silica particles in Experimental Examples 2-1-(1) and (9) and the microporous silica particles in Experimental Example 2-1-(1) as a control were measured according to Equation 1 above, and results thereof are shown in FIG. 11 (SBF used as the suspension and the solvent).

Referring to FIG. 11, it can be seen that the porous silica particles of the inventive example have a significantly larger t than the control.

(4) pH

Absorbance of the porous silica particles in Experimental Example 2-1-(4) for each pH was measured. The absorbance was measured in SBF and in Tris at pH 2, 5, and 7.4, and results thereof are shown in FIG. 12.

Referring to FIG. 12, it could be seen that, although there is a difference in t in relation to pH, t at which all absorbance ratio becomes 1/2 was 24 or more.

(5) Charging

Absorbance of the porous silica particles in Experimental Example 2-2-(1)-1 was measured, and results thereof are shown in FIG. 13 (Tris (pH 7.4) used as the suspension and the solvent).

Referring to FIG. 13, it could be seen that t at which the absorbance ratio of the positively charged particles becomes 1/2 was 24 or more.

**2-5. Release of supported nucleic acid molecules**

10 $\mu$l of porous silica particles loaded with Cy5-siRNA were resuspended in SBF (pH 7.4, 37 °C) and put into a permeable membrane with a pore diameter of 20 kDa (a tube in FIG. 14).

Thereafter, the permeation tube was immersed in 1.5 ml of SBF.

Release of siRNA was performed at 37 °C under 60 rpm horizontal stirring.

Before 24 hours, the discharged solvent was recovered at 0.5, 1, 2, 4, 8, 12, and 24 hours lapse, and thereafter, 0.5 ml of the discharged solvent was recovered at 24 hours interval for fluorescence measurement, followed by adding equal amount of SBF.

Fluorescence intensity of Cy5-siRNA was measured at 670 nm wavelength ($\lambda_{ex}$ = 647 nm) to determine a degree of emission of siRNA, and results thereof are shown in FIG. 15.

Referring to FIG. 15, it can be seen that a time of 50% siRNA release is about 48 hours.

**3. In *vitro* LEM-siTSLP treatment results**

**3-1. Identification of HaCaT cell and HeLa intracellular morphology**

(1) Experimental method

HaCaT cells or HeLa cells were seeded in an 8-well chamber (Lab-Tek Chamber slide system) by $2.0 \times 10^3$ cells and then incubated for 24 hours. After washing the cells twice with 1 $\times$ PBS, TAMRA fluorescence-labeled siRNA (50 ng) was loaded onto FITC fluorescence-labeled DDV (1 $\mu$g) to prepare siRNA and DDV complex, followed by treating the cells with the above complex in a serum-free medum for 2 hours.

The DDV used herein was the porous silica particles of Example 2-2-(1)-2 -②, and the siRNA used herein was siRNA #1.

After 2 hours, the cells were washed twice with 1 $\times$ PBS. Subsequently, the medium was replaced with 10% FBS containing medium, followed by nuclear staining the cells (2, 6, 8, 12, 18, 24 hours) in the order of time using Hoechst 33342 (Invitrogen). Then, change in morphology of the cells and intracellular distribution of siRNA and DDV in the cells over time were observed by Delta Vision Elite High Resolution Microscope (GE Healthcare Life Sciences) using a 60 x lens.

(2) Experimental result

After treatment of the cells using the siRNA and DDV complex, it was found at the early stage of about 2 hours that orange to yellow fluorescence appeared mostly in the cells. This is expected because red fluorescence-labeled siRNA supported on green fluorescence-labeled DDV is introduced into the cells so that green fluorescence and red fluorescence overlap to appear orange to yellow fluorescence in the cells.

Over time, it was confirmed that orange to yellow fluorescence considerably disappeared while predominantly exhibiting green fluorescence. This is expected because siRNA is released from DDV, such that orange to yellow fluorescence disappeared over time, while predominantly exhibiting green fluorescence of siRNA.

From the above results, it was determined that the DDV and siRNA complex is well introduced into the cells, and the siRNA drug can be released into the cells in a sustained manner.

### 3-2. In *vitro* identification of TSLP mRNA knockdown by LEM-siTSLP

In order to measure target gene knockdown efficiency of LEM-siTSLP, HaCaT (human keratinocyte cells) was subjected to the following experiment using LEM-siTSLP #1, LEM-siTSLP #14 and LEM-siTSLP #21 prepared by the above-described method.

Before proceeding with the TSLP mRNA knockdown identification experiment, it was confirmed that TSLP expression is induced by treating HaCaT cells with SLLPRL (see FIG. 18).

First, HaCaT cells were treated with the above three types of LEM-siTSLP (25 pmol) and then incubated with 200 vM SLIGRL in order to induce TSLP expression. As a result, LEM-siTSLP treatment on the cell-line has reduced the mRNA expression level of TSLP and, in particular, LEM-siTSLP #1 treatment showed significant effects on inhibition of mRNA expression of TSLP.

Meanwhile, the controls (siTSLP #1, siTSLP #14, siTSLP #21 only) were treated with siRNA only, and did not show effects of inhibiting mRNA expression of TSLP in HaCaT cells (see FIG. 19). These results indicate that, unlike the controls, LEM-siTSLP may efficiently transfect siTSLP into cells and induce knockdown of the TSLP gene.

### 4. In *vitro* sustained siTSLP release of LEM-siTSLP

In the present experiment, LEM-siTSLP was confirmed to maintain TSLP knockdown effects longer than LNP in HaCaT cells.

HaCaT cells were treated with LEM-siTSLP #1 (25 pmol) and siTSLP #1 (25 pmol) supported on LNP, followed by SLIGRL treatment to induce TSLP expression.

As the DDV carrying siTSLP #1, the porous silica particles of Example 2-2-(1)-2) -② were used.

Inhibition of TSLP expression in HaCaT cells was continued up to 96 hours after LEM-siTSLP #1 treatment (TSLP expression level, 72 hours: 15%, 96 hours: 22%), but TSLP expression inhibition efficiency of siTSLP #1 loaded on LNP was not high at both 72 and 96 hours (TSLP expression level, 72 hours: 43%, 96 hours: 56%) (see FIG. 20). That is, it can be seen that LEM-siTSLP inhibits target mRNA expression at a high level for a longer period of time than siTSLP loaded on LNP in cells.

### 5. Delivery of LEM-siTSLP and *ex-vivo* analysis of change in distribution of LEM-siTSLP

C57BL/6 mouse buccal tissues were subjected to injection of fluorescent-labeled LEM-siTSLP consisting of FITC-conjugated siTSLP loaded on TAMRA-conjugated DegradaBALL, whereas only unsupported FITC-conjugated siTSLP was injected through a subcutaneous infusion route. Then, durations at the injection sites of LEM-siTSLP and siTSLP, respectively, were compared. The present experiment confirmed LEM-siTSLP delivery effect and change in distribution of the same.

The porous silica particles of Example 2-2-(1)-2)-② were used as DDV and the siTSLP used herein was siRNA #1.

Fluorescent image analysis of the resected mouse buccal skin and fragmented buccal skin was performed on days 1, 2 and 4 after injection. Fluorescence of TAMRA-DegradaBALL carrying FITC-siTSLP showed strong luminescence at the injection site on day 1. The fluorescence is slowly decreased over time but the fluorescence at the injection site is still strongly remained until 4 days after the injection (see FIGS. 21 and 23). A tendency of decreasing fluorescence at the injection site over time corresponded to a skin section sliding tendency. On the other hand, no fluorescence signal was observed in the excised skin or fragmented skin slides from mice injected with only unsupported FITC-siTSLP. This suggested that siTSLP is rapidly dispersed in the body or is degraded into small pieces to induce very rapid diffusion when only siTSLP without DDV is administered (see FIGS. 22 and 23). From the data, it could be seen that the skin with treatment of LEM-siTSLP has maintained a significantly higher concentration level of siTSLP than the case of treatment with siTSLP not supported on DDV, for at least 4 days after the injection.

### 6. *In-vivo* analysis of TSLP knockdown effect by LEM-siTSLP injection

TSLP knockdown effects in mice injected with LEM-siTSLP were investigated by mouse behavioral analysis.

After clearly removing hair of the mouse buccal tissues, PBS and DDV loaded with siSLP (0.7 nmol siRNA, 150 ug BALL in 20 uL PBS) were intradermally injected (ID) on the right buccal part, respectively.

The porous silica particles of Example 2-2-(1)-2-② were used as DDV, and siTSLP used herein was a combination of the sense RNA sequence (5'-CGAGCAAAUCGAGGACUGUdTdT-3' (SEQ ID NO: 124)) and the antisense RNA sequence (5'-ACAGUCCUCGAUUUGCUCGdTdT-3' (SEQ ID NO: 125)).

After 48 hours of injection, TSLP induction was performed by ID injection of 100 μg (in 20 μl PBS) of SLIGRL peptide, and scratching behavior was observed for 30 minutes immediatedly after injection in order to compare behavior conditions.

Specifically, the number of times of scratching a buccal part by a mouse was analyzed and, in order to distinguish the scratching from the grooming behavior, only the number of times of scratching the cheek with the 'hind foot' of the mouse was counted. Raising then putting down the hind foot counts as one time but, when continuously scratching for 1 second or more without putting down the hind foot, the duration was measured and counted once per second.

FIG. 24 shows the total number of times of scratching measured during 30 minutes, and FIG. 25 shows the number of times of scratching measured at 5 minute interval.

**Claims**

1. A pharmaceutical composition for preventing or treating atopic diseases, comprising:

   porous silica particles carrying nucleic acid molecules that complementarily bind to at least a portion of TSLP mRNA,
   wherein the porous silica particles are **characterized in that** t, at which an absorbance ratio in the following Equation 1 becomes 1/2, is 24 or more,

   $$[\text{Equation 1}]$$

   $$A_t/A_0$$

   (wherein $A_0$ is absorbance of the porous silica particles measured by putting 5 ml of suspension containing 1 mg/ml of porous silica particles into a cylindrical permeable membrane having pores with a pore diameter of 50 kDa,
   15 ml of the same solvent as the suspension comes into contact with an outside of the permeable membrane, and the inside/outside of the permeable membrane are horizontally stirred at 60 rpm and at 37 °C,
   pH of the suspension is 7.4, and
   $A_t$ indicates absorbance of the porous silica particle measured after lapse of "t" hours since $A_o$ was measured).

2. The pharmaceutical composition according to claim 1, wherein the porous silica particles are prepared by: reacting the silica particles having pores of less than 5 nm in diameter with a swelling agent at 120 to 180 °C for 24 to 96 hours to expand the pores of less than 5 nm in diameter; and calcining the pores of the expanded silica particles at a temperature of 400 °C or higher for 3 hours or more.

3. The pharmaceutical composition according to claim 1, wherein an average diameter of the porous silica particles ranges from 150 to 1000 nm, a BET surface area ranges from 200 to 700 m$^2$/g, and a volume per gram ranges from 0.7 to 2.2 ml.

4. The pharmaceutical composition according to claim 1, wherein the nucleic acid molecule is one of siRNA, dsRNA, PNA or miRNA.

5. The pharmaceutical composition according to claim 4,
   wherein the nucleic acid molecules includes at least one siRNA or dsRNA selected from the group consisting of: siRNA composed of a sense RNA having a sequence of SEQ ID NO: 1 and an antisense RNA having a sequence of SEQ ID NO: 47; dsRNA composed of a strand having a sequence of SEQ ID NO: 24 and another strand complementary thereto; siRNA composed of a sense RNA having a sequence of SEQ ID NO: 2 and an antisense RNA having a sequence of SEQ ID NO: 48; dsRNA composed of a strand having a sequence of SEQ ID NO: 25 and another strand complementary thereto; siRNA composed of a sense RNA having a sequence of SEQ ID NO: 3 and an antisense RNA having a sequence of SEQ ID NO: 49; dsRNA composed of a strand having a sequence of SEQ ID NO: 26 and another strand complementary thereto; siRNA composed of a sense RNA having a sequence of SEQ ID NO: 4 and an antisense RNA having a sequence of SEQ ID NO: 50; dsRNA composed of a strand having a sequence of SEQ ID NO: 27 and another strand complementary thereto; siRNA composed of a sense RNA having a sequence of SEQ ID NO: 5 and an antisense RNA having a sequence of SEQ ID NO: 51; dsRNA composed of a strand having a sequence of SEQ ID NO: 28 and another strand complementary thereto; siRNA composed of a sense RNA having a sequence of SEQ ID NO: 6 and an antisense RNA having a sequence of SEQ ID NO: 52; dsRNA composed of a strand having a sequence of SEQ ID NO: 29 and another strand complementary thereto; siRNA composed of a sense RNA having a sequence of SEQ ID NO: 7 and an antisense RNA having a sequence of SEQ ID NO: 53; dsRNA composed of a strand having a sequence of SEQ ID NO: 30 and another strand complementary thereto; siRNA composed of a sense RNA having a sequence of SEQ ID NO: 8 and an antisense RNA having a sequence of SEQ ID NO: 54; dsRNA composed of a strand having a sequence of SEQ ID NO: 31 and another strand complementary thereto; siRNA composed of a sense RNA having a sequence of SEQ ID NO: 9 and an antisense RNA having a sequence of SEQ ID NO: 55; dsRNA composed of a strand having a sequence of SEQ ID NO: 32 and another strand complementary thereto; siRNA composed of a sense RNA having a sequence of SEQ ID NO: 10 and an antisense RNA having a sequence of SEQ ID NO: 56; dsRNA composed of a strand having a sequence of SEQ ID NO: 33 and another strand complementary thereto; siRNA composed of a sense RNA having a sequence of SEQ ID NO: 11 and an antisense RNA having a sequence of SEQ ID NO: 57; dsRNA composed of a strand having a sequence of SEQ ID NO: 34 and another strand complementary thereto; siRNA composed of a sense RNA having a sequence of SEQ ID NO: 12 and an antisense RNA having a sequence of SEQ ID NO: 58; dsRNA composed of a strand having a sequence of SEQ ID NO: 35 and another strand complementary thereto; siRNA composed of a sense RNA having a sequence of SEQ ID NO: 13 and an antisense RNA having a sequence of SEQ ID NO: 59; dsRNA composed of a strand having a sequence of SEQ ID NO: 36 and another strand complementary thereto; siRNA composed of a sense RNA having a sequence of SEQ ID NO: 14 and an antisense RNA having a sequence of SEQ ID NO: 60; dsRNA composed of a strand having a sequence of SEQ ID NO: 37 and another strand complementary thereto; siRNA composed of a sense RNA having a sequence of SEQ ID NO: 15 and an antisense RNA having a sequence of SEQ ID NO: 61; dsRNA composed of a strand having a sequence of SEQ ID NO: 38 and another strand complementary thereto; siRNA composed of a sense RNA having a sequence of SEQ ID NO: 16 and an antisense RNA having a sequence of SEQ ID NO: 62; dsRNA composed of a strand having a sequence of SEQ ID NO: 39 and another strand complementary thereto; siRNA composed of a sense RNA having a sequence of SEQ ID NO: 17 and an antisense RNA having a sequence of SEQ ID NO: 63; dsRNA composed of a strand having a sequence of SEQ ID NO: 40 and another strand complementary thereto; siRNA composed of a sense RNA having a sequence of SEQ ID NO: 18 and an antisense RNA having a sequence of SEQ ID NO: 64; dsRNA composed of a strand having a sequence of SEQ ID NO: 41 and another strand complementary thereto; siRNA composed of a sense RNA having a sequence of SEQ ID NO: 19 and an antisense RNA having a sequence of SEQ ID NO: 65; dsRNA composed of a strand having a sequence of SEQ ID NO: 42 and another strand complementary thereto; siRNA composed of a sense RNA having a sequence of SEQ ID NO: 20 and an antisense RNA having a sequence of SEQ ID NO: 66; dsRNA composed of a strand having a sequence of SEQ ID NO: 43 and another strand complementary thereto; siRNA composed of a sense RNA having a sequence of SEQ ID NO: 21 and an antisense RNA having a sequence of SEQ ID NO: 67; dsRNA composed of a strand having a sequence of SEQ ID NO: 44 and another strand complementary thereto; siRNA composed of a sense RNA having a sequence of SEQ ID NO: 22 and an antisense RNA having a sequence of SEQ ID NO: 68; dsRNA composed of a strand having a sequence of SEQ ID NO: 45 and another strand complementary thereto; siRNA composed of a sense RNA having a sequence of SEQ ID NO: 23 and an antisense RNA having a sequence of SEQ ID NO: 69; and dsRNA composed of a strand having a sequence of SEQ ID NO: 46 and another strand complementary thereto.

6. The pharmaceutical composition according to claim 5, further comprising a sequence of UU at 3'-terminals of the sense RNA and the antisense RNA sequence.

7. The pharmaceutical composition according to claim 5, further comprising a sequence of dTdT at 3'-terminals of the sense RNA and the antisense RNA sequence.

8. The pharmaceutical composition according to claim 5, wherein the nucleic acid molecule is at least one siRNA or dsRNA selected from the group consisting of: siRNA composed of a sense RNA having the sequence of SEQ ID NO: 1 and an antisense RNA having the sequence of SEQ ID NO: 47; dsRNA composed of a strand having the sequence of SEQ ID NO: 24 and another strand complementary thereto; siRNA composed of a sense RNA having the sequence of SEQ ID NO: 14 and an antisense RNA having the sequence of SEQ ID NO: 60; dsRNA composed of a strand having the sequence of SEQ ID NO: 37 and another strand complementary thereto; siRNA composed of a sense RNA having the sequence of SEQ ID NO: 21 and an antisense RNA having the sequence of SEQ ID NO: 67; and dsRNA composed of a strand having a sequence of SEQ ID NO: 44 and another strand complementary thereto.

9. The pharmaceutical composition according to claim 8, wherein the nucleic acid molecule include the siRNA composed of a sense RNA having the sequence of SEQ ID NO: 1 and an antisense RNA having the sequence of SEQ ID NO: 47, or the dsRNA composed of a strand having the sequence of SEQ ID NO: 24 and a complementary thereof.

10. The pharmaceutical composition according to claim 1, wherein the porous silica particles are positively charged at neutral pH on an outer surface thereof or an inside of the pores.

11. The pharmaceutical composition according to claim 1, wherein the porous silica particles have hydrophilic or hydrophobic functional groups.

12. The pharmaceutical composition according to claim 1, wherein the atopic disease is at least one selected from the group consisting of bronchial asthma, allergic rhinitis, urticaria, atopic dermatitis, allergic conjunctivitis, allergic dermatitis, allergic contact dermatitis, inflammatory skin disease, pruritus and food allergy.

[FIG. 1]

## SLIGRL peptide synthesis

[M+H]⁺ = 657.319

[M+Na]⁺ = 679.29

| Peptide Sequence: | SLIGRL |
|---|---|
| Number of Residues: | 6 |
| N-terminus: | H |
| C-terminus: | NH₂ |
| Average Weight: | 656.83 |
| Monoisotopic Weight: | 656.44 |

[FIG. 2]

[FIG. 3]

[FIG. 4]

[FIG. 5]

2 L scale                    10 L scale

[FIG. 6]

[FIG. 7]

[FIG. 8]

DDV solution

Dialysis membrane

Fresh SBF

[FIG. 9]

t(DC$_{50}$) = ~2.4 days (~58 hr) (±20%)
t(DC$_{80}$) = ~3.9 days (~93 hr) (±20%)
t(DC$_{95}$) = ~7 days (~168 hr) (±20%)

r$^2$ (t(DC$_0$)~t(DC$_{80}$))= 0.97

t(DC$_{50}$)

t(DC$_{80}$)

t(DC$_{95}$)

[FIG. 10]

| Sample | $t_{50\%}$ |
|---|---|
| DDV(200)$_{10}$ | 57.4 h |
| DDV(500)$_{10}$ | 37.5 h |
| DDV(1000)$_{10}$ | 30.0 h |

[FIG. 11]

| Samples | $t_{50\%}$ |
|---|---|
| MSN(200)$_2$ | 17.9 h |
| DDV(200)$_{10}$ | 57.4 h |
| DDV(200)$_{17}$ | 53.6 h |

[FIG. 12]

DDV(300)$_{17}$

[FIG. 13]

DDV(300)$_{17}$–NH$_2$

t$_{50\%}$ = about 2.5 days

[FIG. 14]

mini-dialysis tube

siRNA/**pSP** suspension

dialysis membrane

medium (PBS)

ep-tube

[FIG. 15]

[FIG. 16]

**HaCaT (turtle shell-like morphology)**

[FIG. 17]

**HeLa (epithelial morphology)**

[FIG. 18]

[FIG. 19]

[FIG. 20]

[FIG. 21]

## FITC-siRNA +TAMRA-DDV

[FIG. 22]

# FITC-siRNA only

Day 1  Day 2  Day 4

Merged

TAMRA

FITC

DAPI

[FIG. 23]

Day 1 — FITC, TAMRA; Day 2 — FITC, TAMRA; Day 4 — FITC, TAMRA

FITC-siRNA + TAMRA-DDV

FITC-siRNA only

[FIG. 24]

[FIG. 25]

[FIG. 26]

## INTERNATIONAL SEARCH REPORT

| International application No. |
| --- |
| **PCT/KR2019/095028** |

**A. CLASSIFICATION OF SUBJECT MATTER**

*A61K 9/14(2006.01)i, A61K 47/02(2006.01)i, A61K 31/7088(2006.01)i, A61P 37/08(2006.01)i, A61P 11/06(2006.01)i, C12N 15/113(2010.01)i, C01B 33/12(2006.01)i*

According to International Patent Classification (IPC) or to both national classification and IPC

**B. FIELDS SEARCHED**

Minimum documentation searched (classification system followed by classification symbols)

A61K 9/14; A61K 39/395; A61K 48/00; A61K 8/60; A61K 9/16; A61P 17/00; A61K 47/02; A61K 31/7088; A61P 37/08; A61P 11/06; C12N 15/113; C01B 33/12

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: TSLP(thymic stromal lymphopoietin), atopy, siRNA, porous silica

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2016-0016475 A (WON, Cheolhee et al.) 15 February 2016 See abstract; table 1; claims 1-6; paragraph [0065]. | 1,4-9,12 |
| Y | | 2-3,10-11 |
| Y | KR 10-2016-0011565 A (LEMONEX INC. et al.) 01 February 2016 See abstract; paragraphs [0067], [0088], [0093]-[0096]; table 3; claims 1, 5, 14, 17. | 2-3,10-11 |
| X | KR 10-2017-0029449 A (WON, Cheolhee et al.) 15 March 2017 See abstract; table 1; claims 1-5. | 1,4-9,12 |
| Y | | 2-3,10-11 |
| X | KR 10-2017-0057194 A (LEMONEX INC. et al.) 24 May 2017 See abstract; paragraph [0013]; table 1; claims 1-4. | 1,4-9,12 |
| Y | | 2-3,10-11 |
| Y | NA, H.-K. et al. Efficient functional delivery of siRNA using mesoporous silica nanoparticles with ultralarge pores. Small. 2012, vol. 8, no. 11, pages 1752-1761 See abstract; figures 1-2; pages 1753-1754. | 2-3,10-11 |

☐ Further documents are listed in the continuation of Box C.   ☒ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 NOVEMBER 2019 (15.11.2019) | **15 NOVEMBER 2019 (15.11.2019)** |

| Name and mailing address of the ISA/KR | Authorized officer |
| --- | --- |
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/095028**

| Patent document cited in search report | Publication date | Patent family member | Publication date |
|---|---|---|---|
| KR 10-2016-0016475 A | 15/02/2016 | None | |
| KR 10-2016-0011565 A | 01/02/2016 | CN 107072951 A | 18/08/2017 |
| | | EP 3173074 A1 | 31/05/2017 |
| | | EP 3173074 A4 | 07/03/2018 |
| | | JP 2017-529384 A | 05/10/2017 |
| | | JP 6426288 B2 | 21/11/2018 |
| | | KR 10-1799557 B1 | 20/11/2017 |
| | | US 2017-0172923 A1 | 22/06/2017 |
| | | WO 2016-013751 A1 | 28/01/2016 |
| KR 10-2017-0029449 A | 15/03/2017 | KR 10-1841712 B1 | 23/03/2018 |
| KR 10-2017-0057194 A | 24/05/2017 | KR 10-1841713 B1 | 23/03/2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REMINGTON.** Pharmaceutical Science. Mack Publishing Company